# EUROPEAN PATENT APPLICATION

(11) **EP 4 810 513 A1**
(43) Date of publication of application: **23.09.2026**
(21) Application number: 24891802.1
(22) Date of filing: 15.11.2024
(51) Int. Cl.: C07K 16/18, A61P 35/00, A61K 38/00, A61K 39/00

(54) **SINGLE DOMAIN ANTIBODY SPECIFICALLY BINDING TO SOX2, FUSION PROTEIN INCLUDING SAME, AND USE THEREOF**

(30) Priority: 15.11.2023 KR 20230158319
(71) Applicant: Genexine, Inc., Seoul 07789 (KR)
(72) Inventor: YONG, Yeryoung, Seoul 06544 (KR); LEE, Seunghyun, Seoul 02720 (KR); CHOI, Jaehyun, Seoul 07360 (KR); LIM, Saeyi, Seoul 07622 (KR); SONG, Hyerin, Seoul 07599 (KR); BAI, Xuelian, Goyang-si Gyeonggi-do 10323 (KR); OH, Yeonji, Seongnam-si Gyeonggi-do 13260 (KR)
(74) Representative: dompatent
(86) International application number: PCT/KR2024/018064
(87) International publication number: WO 2025/105856

(57) **Abstract**

The present invention relates to a single domain antibody specifically binding to SOX2 via E3 ubiquitin ligase or an antigen-binding fragment thereof, a fusion protein for enhancing proteolysis using same, and a use thereof. The fusion protein including the anti-SOX2 single domain antibody and the E3 ubiquitin ligase fragment of the present invention specifically binds to SOX2 to degrade SOX2. SOX2 degradation by the fusion protein promotes growth inhibition and apoptosis of cancer cell lines. In addition, when used in combination with an anticancer agent, the fusion protein inhibited the growth of cancer cells that exhibit drug resistance. The fusion protein inhibits tumor growth in a tumor mouse model. Therefore, the fusion protein of the present invention can be advantageously used for preventing or treating cancer.

## Description

### TECHNICAL FIELD

The present invention relates to a single domain antibody or an antigen-binding fragment thereof that specifically binds to SOX2 by E3 ubiquitin ligase, a fusion protein using the antibody or the antigen-binding fragment thereof for enhancing protein degradation, and the use of the fusion protein. Specifically, the present invention relates to the use of an anti-SOX2 single domain antibody targeting SOX2 or an antigen-binding fragment thereof and a fusion protein including an E3 ubiquitin ligase fragment and a variant thereof in the prevention or treatment of a SOX2-mediated disease.

### BACKGROUND ART

To date, new drugs have been developed based on the principle of inhibiting the active site or ligand binding site of target proteins for diseases. Of the approximately 20,000 human proteins, only about 3% of proteins are known as FDA-approved drug targets, and about 3,100 proteins (16%), which correspond to most disease target proteins, are considered inapplicable to current new drug development technology. In addition, existing active site-directed new drugs often report problems with drug resistance.

PROTAC (proteolysis-targeting chimera) is a dual-binding molecule that binds to disease-causing proteins and induces proteolysis via the proteasome. PROTAC can induce the degradation of proteins (induced proteolysis) that cannot be degraded by existing technologies by using the ubiquitin-proteasome system (UPS), which is an endogenous protein degradation mechanism that cells use to degrade proteins. Therefore, it is attracting attention as a new paradigm for overcoming the limitations of traditional new drug development.

PROTAC is composed of two protein binding molecules, and the proteins bind to E3 ubiquitin ligase and target protein, respectively. Through this binding, PROTAC brings the target protein close to the E3 ligase, thereby inducing ubiquitination. Ubiquitination involves three steps of activation, conjugation and ligation, which are performed by ubiquitin-activating enzyme (E1), ubiquitin-conjugating enzyme (E2) and ubiquitin ligase (E3). As a result of this cascade, ubiquitin is covalently bonded to the target protein, and the ubiquitinated protein is degraded by the proteasome.

The PROTAC technology was first introduced in 2001, and is currently receiving much attention and actively developing related technologies. Most of the PROTAC drugs have been developed by being composed of small molecular compounds. Although the known PROTAC drugs are very useful, improvements are needed to address the inherent low solubility of small molecular compounds, low protein degradation efficiency due to decreased intracellular penetration and safety concerns, and there is a need for PROTAC drugs that improve these issues.

### DISCLOSURE

### TECHNICAL PROBLEM

Accordingly, the inventors of the present invention conducted research to develop a PROTAC that specifically targets SOX2 (SRY-box transcription factor 2) and induces the degradation of SOX2, and as a result, it was confirmed that a fusion protein including a novel anti-SOX2 single domain antibody and a fragment of E3 ubiquitin ligase specifically targets SOX2 and promotes its degradation, thereby completing the present invention.

### TECHNICAL SOLUTION

In order to achieve the above object, one aspect of the present invention provides an anti-SOX2 single domain antibody or an antigen-binding fragment thereof, including a heavy chain variable region including HCDR1 including any one amino acid sequence selected from the group consisting of SEQ ID NO: 56, SEQ ID NO: 63, SEQ ID NO: 69, SEQ ID NO: 73, SEQ ID NO: 78, SEQ ID NO: 82, SEQ ID NO: 87 and SEQ ID NO: 94; HCDR2 including any one amino acid sequence selected from the group consisting of SEQ ID NO: 58, SEQ ID NO: 65, SEQ ID NO: 71, SEQ ID NO: 72, SEQ ID NO: 74, SEQ ID NO: 75, SEQ ID NO: 76, SEQ ID NO: 79, SEQ ID NO: 84 and SEQ ID NO: 89; and HCDR3 including any one amino acid sequence selected from the group consisting of SEQ ID NO: 60, SEQ ID NO: 67, SEQ ID NO: 81, SEQ ID NO: 85, SEQ ID NO: 91 and SEQ ID NO: 96.

Another aspect of the present invention provides a polynucleotide encoding the antibody or antigen-binding fragment thereof, an expression vector loaded with the polynucleotide, and a transformed cell into which the expression vector is introduced.

Still another aspect of the present invention provides a method for producing an anti-SOX2 single domain antibody or an antigen-binding fragment thereof, including culturing the transformed cell and obtaining the antibody or antigen-binding fragment thereof from the culture medium.

Still another aspect of the present invention provides a fusion protein including the antibody or antigen-binding fragment thereof; and a fragment of E3 ubiquitin ligase or a variant thereof.

Still another aspect of the present invention provides a polynucleotide encoding the fusion protein, an expression vector loaded with the polynucleotide and a transformed cell into which the expression vector is introduced.

Still another aspect of the present invention provides a pharmaceutical composition for preventing or treating cancer, including the fusion protein as an active ingredient.

Still another aspect of the present invention provides a method for preventing or treating cancer, including administering the fusion protein, a polynucleotide encoding the fusion protein or a vector loaded with the polynucleotide to a subject.

Still another aspect of the present invention provides the use of the fusion protein, a polynucleotide encoding the fusion protein or a vector loaded with the polynucleotide in the prevention or treatment of cancer.

### ADVANTAGEOUS EFFECTS

The fusion protein including the anti-SOX2 single domain antibody and the E3 ubiquitin ligase fragment of the present invention specifically bound to SOX2 and degraded SOX2. The degradation of SOX2 by the fusion protein inhibited the growth of cancer cell lines and promoted apoptosis. In addition, when the fusion protein was used in combination with an anticancer agent, the growth of cancer cells exhibiting anticancer agent resistance was inhibited. Furthermore, the fusion protein inhibited the growth of tumors in a tumor mouse model. Therefore, the fusion protein of the present invention can be advantageously used for the prevention or treatment of cancer.

### DESCRIPTION OF DRAWINGS

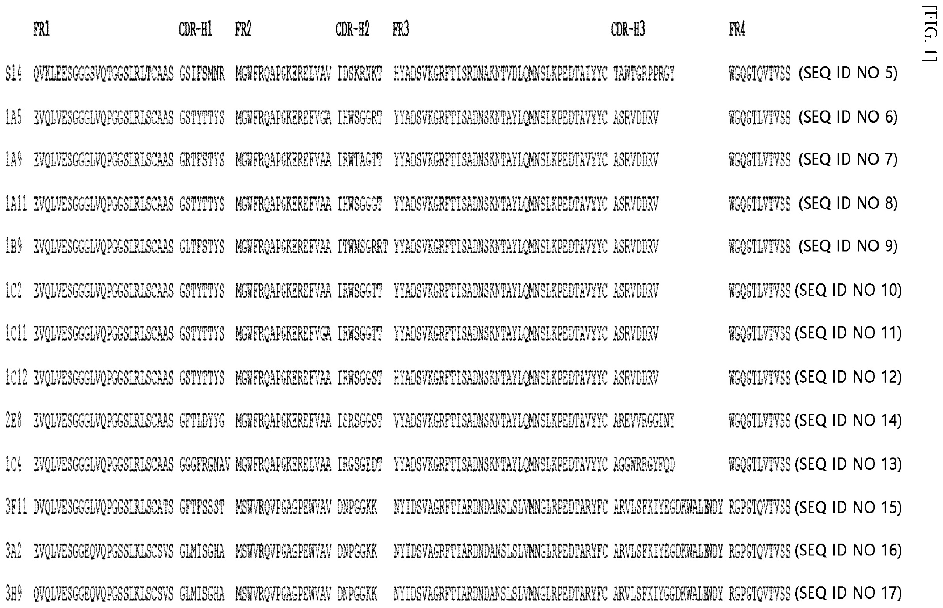

FIG. 1 is a drawing showing the amino acid sequences of 13 anti-huSOX2 VHH antibody clones as one specific example of the present invention.

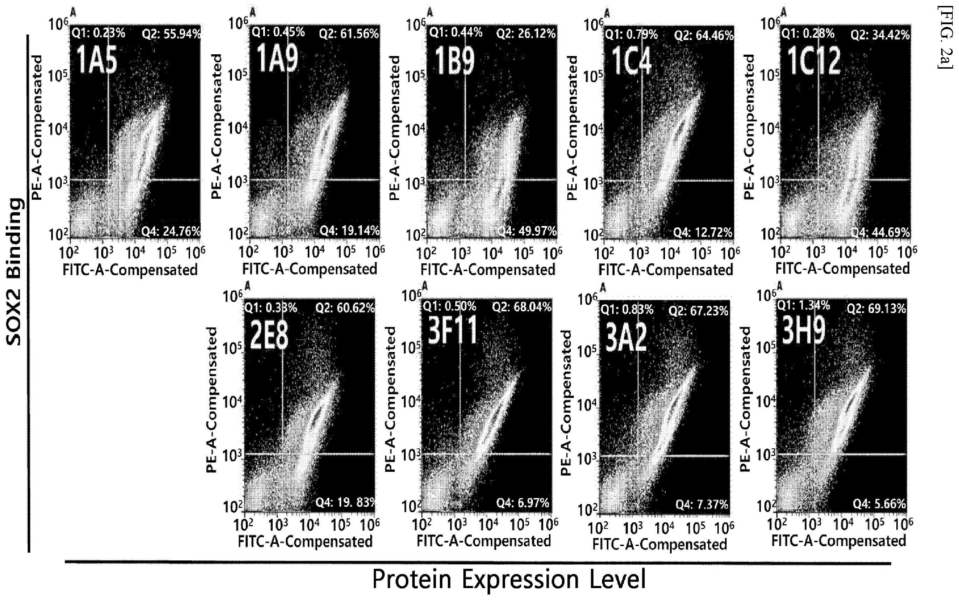

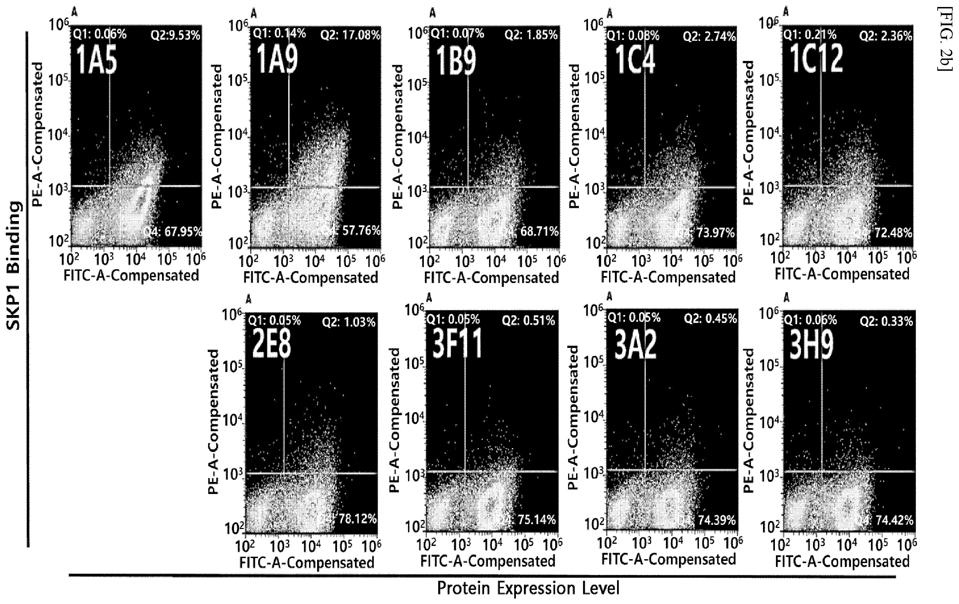

FIGS. 2a and 2b are graphs showing the results of flow cytometry analysis to determine the binding affinity of anti-huSOX2 VHH antibody clones to huSOX2 protein.

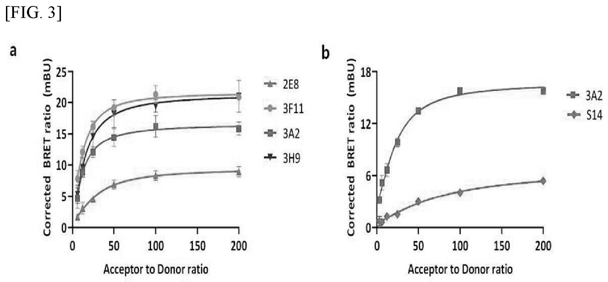

FIG. 3 is a graph showing the results of determining the binding affinity of anti-huSOX2 VHH antibody clones to huSOX2 protein at the cellular level.

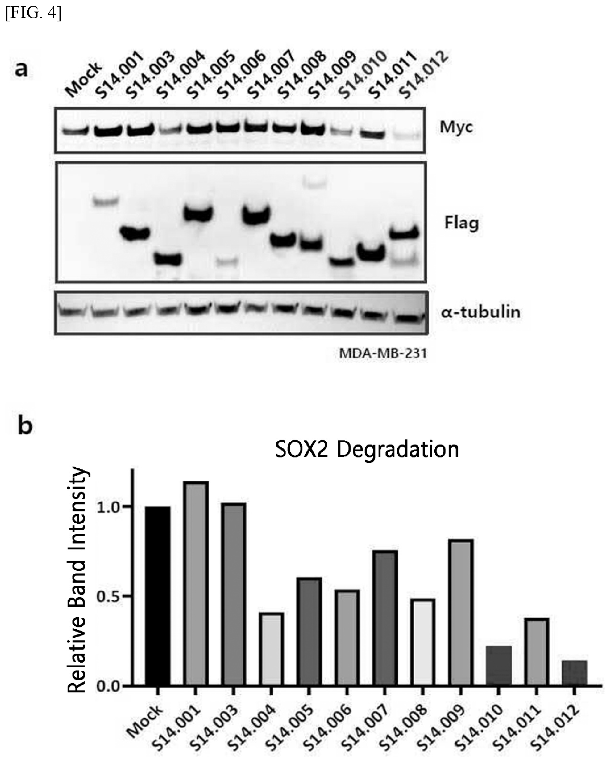

FIG. 4 is a drawing (a) showing the results of confirming the SOX2 protein-specific degradation activity by a fusion protein including S14 (anti-huSOX2 VHH antibody clone) and an E3 ubiquitin ligase fragment, which is one specific example of the present invention, using Western blot, and a graph (b) showing the quantitative results.

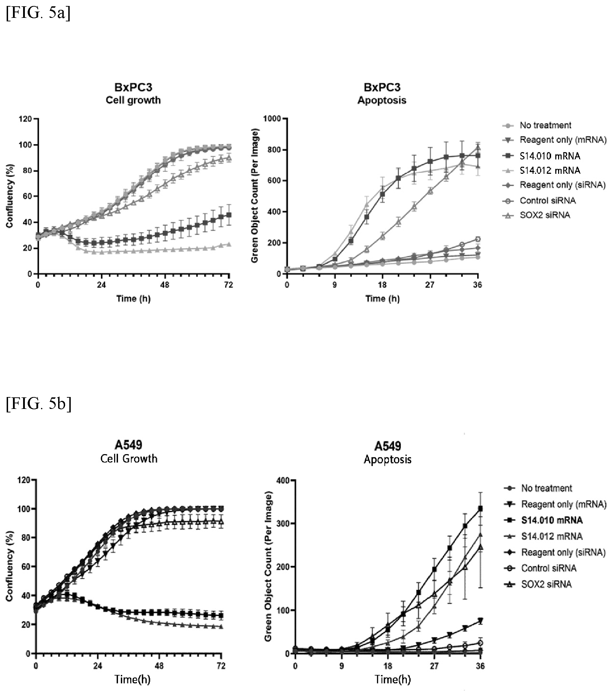

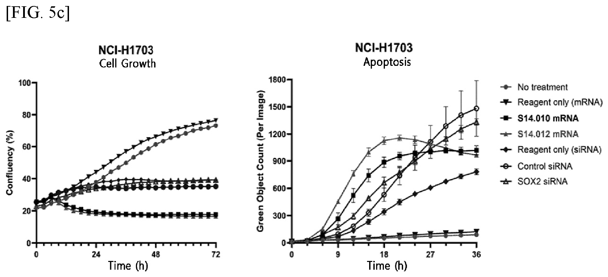

FIGS. 5a to 5c are graphs showing the results of confirming the cell growth inhibition and apoptosis induction effects on cancer cells of mRNA encoding a fusion protein including S14 and E3 ubiquitin ligase fragments, which is one specific example of the present invention.

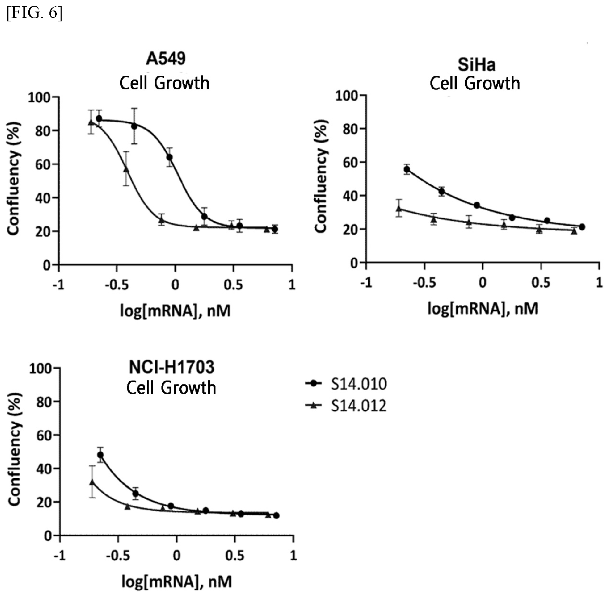

FIG. 6 is a graph showing the results of confirming the cell growth inhibition effects of cancer cells according to concentration-dependent treatment of mRNA encoding a fusion protein including S14 and E3 ubiquitin ligase fragments, which is one specific example of the present invention.

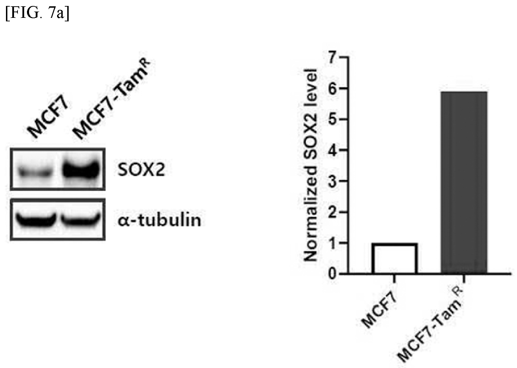

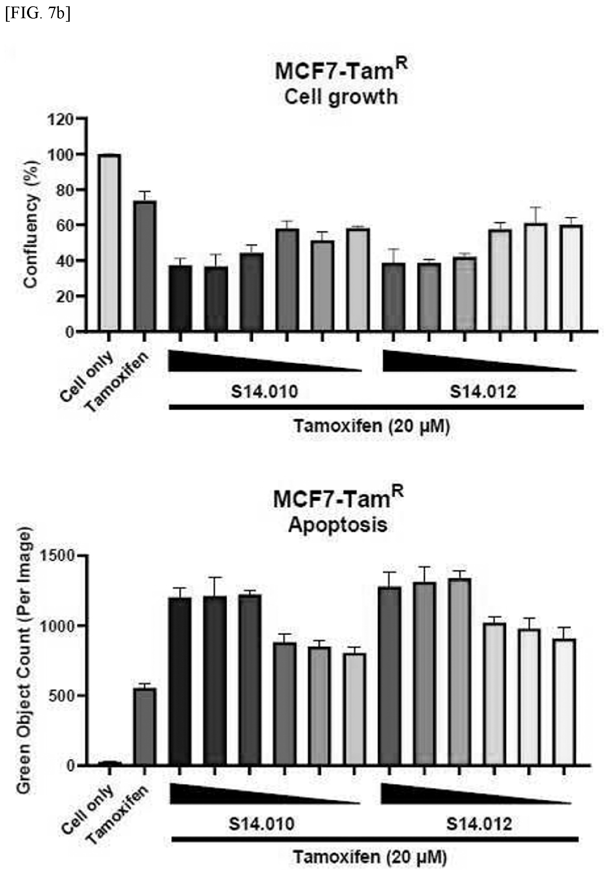

FIGS. 7a and 7b are drawings and graphs showing the results of confirming the expression level of SOX2 (FIG. 7a) and the effects of inducing cell growth and apoptosis by combined use of mRNA encoding a fusion protein including S14 and E3 ubiquitin ligase fragments, which is a specific example of the present invention, and tamoxifen in a tamoxifen-resistant cancer cell line (MCF7-Tam R) (FIG. 7b).

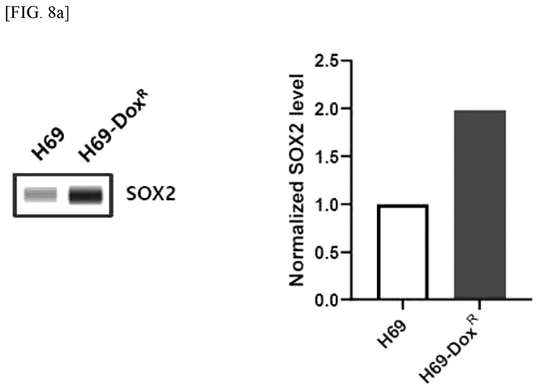

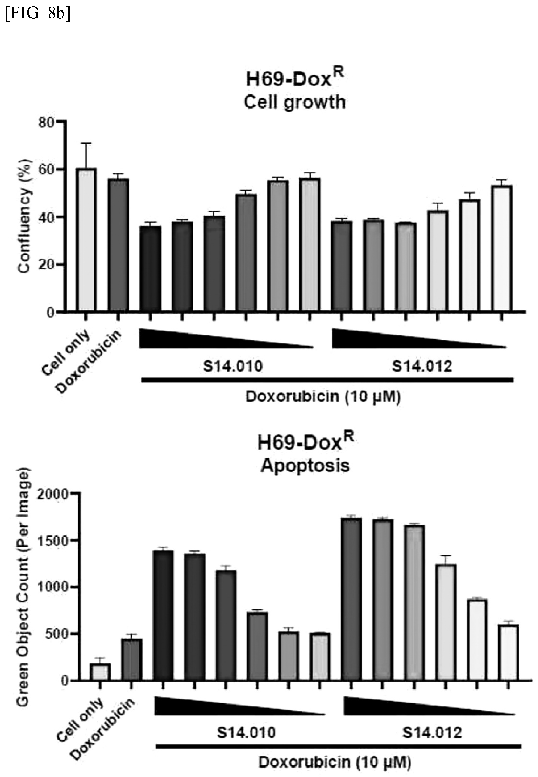

FIGS. 8a and 8b are drawings and graphs showing the results of confirming the expression level of SOX2 (FIG. 8a) and the effects of inducing cell growth and apoptosis by combined use of mRNA encoding a fusion protein including S14 and E3 ubiquitin ligase fragments, which is a specific example of the present invention, and doxorubicin in a doxorubicin-resistant cancer cell line (H69-Dox R) (FIG. 8b).

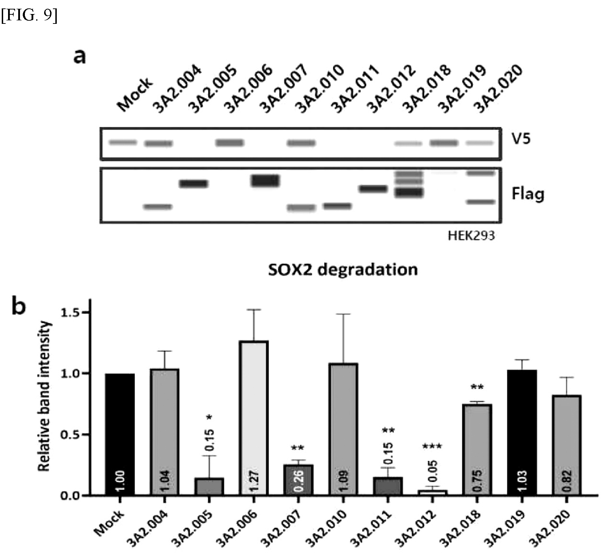

FIG. 9 is a drawing (a) showing the results of confirming the SOX2 protein-specific degradation capability by an expression vector expressing a fusion protein including 3A2 (anti-huSOX2 VHH antibody clone) and an E3 ubiquitin ligase fragment, which is one specific example of the present invention, and a graph (b) showing the quantitative results.

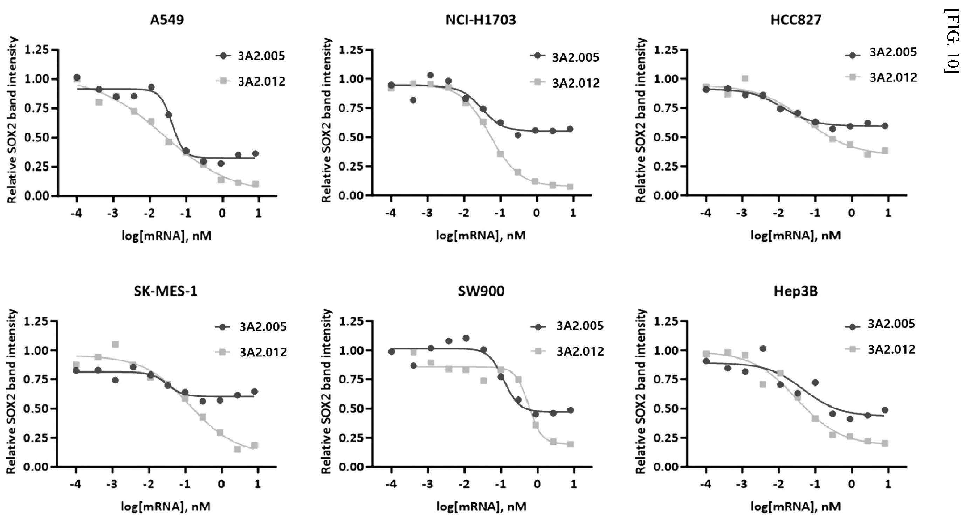

FIG. 10 is a graph showing the results of confirming the SOX2 protein degradation capability by the treatment of mRNA encoding a fusion protein including 3A2 (anti-huSOX2 VHH antibody clone) and an E3 ubiquitin ligase fragment, which is a specific example of the present invention, in human cancer cell lines (lung cancer, liver cancer).

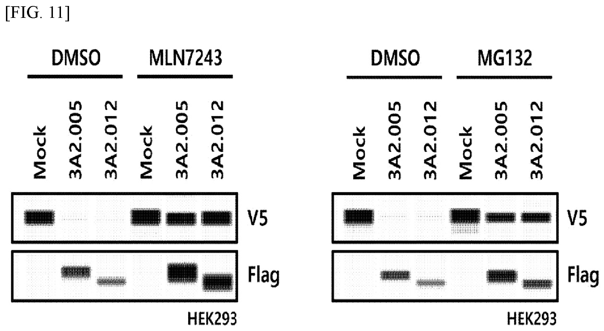

FIG. 11 is a drawing showing the results of confirming whether the SOX2 degradation mechanism is ubiquitin-proteasome dependent using an expression vector expressing a fusion protein including 3A2 (anti-huSOX2 VHH antibody clone) and an E3 ubiquitin ligase fragment, which is one specific example of the present invention.

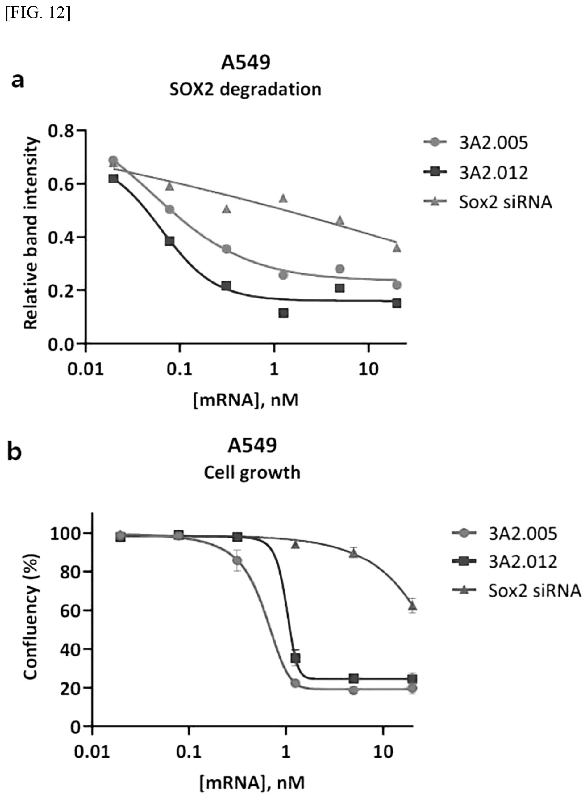

FIG. 12 is a graph showing the results of confirming the effects of inhibiting SOX2 expression and cancer cell growth after the treatment with mRNA encoding a fusion protein including SOX2 siRNA or 3A2 and E3 ubiquitin ligase fragments in a human non-small cell lung cancer cell line (A549 cells).

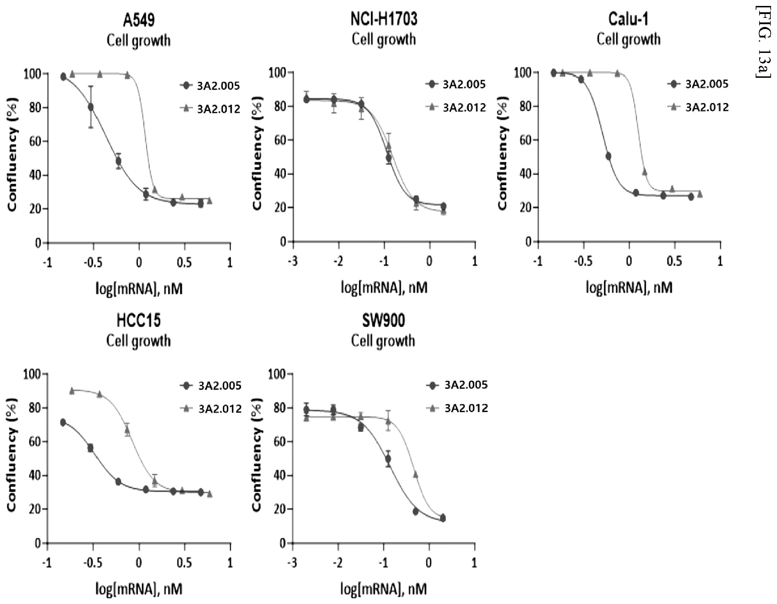

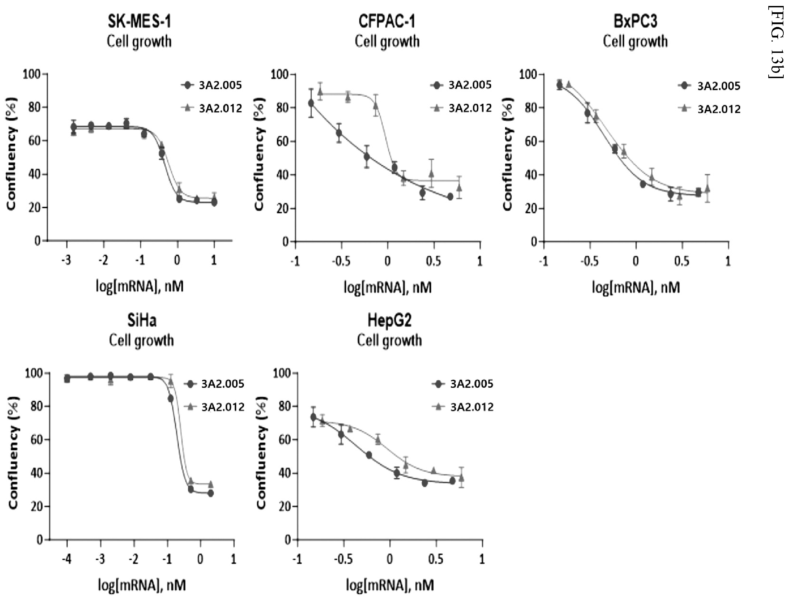

FIGS. 13a and 13b are graphs showing the results of confirming the cell growth inhibition effect by treatment of mRNA encoding a fusion protein including 3A2 (anti-huSOX2 VHH antibody clone) and an E3 ubiquitin ligase fragment, which is a specific example of the present invention, in human cancer cell lines (lung cancer, pancreatic cancer, cervical cancer, and liver cancer).

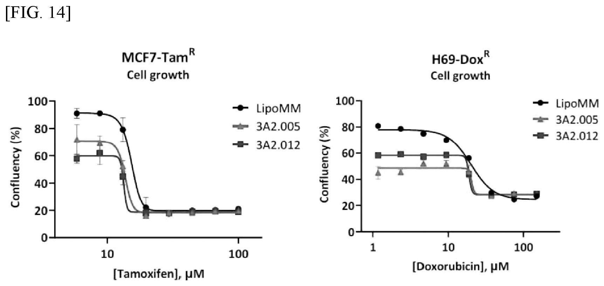

FIG. 14 is a graph showing the results of confirming the cell growth inhibition effect by combined use of mRNA encoding a fusion protein including 3A2 and E3 ubiquitin ligase fragments, which is a specific example of the present invention, and anticancer drugs (tamoxifen or doxorubicin) in each anticancer drug-resistant cancer cell line (MCF7-Tam R, H69-Dox R). LipoMM: Lipofectamine-MessengerMax reagent only.

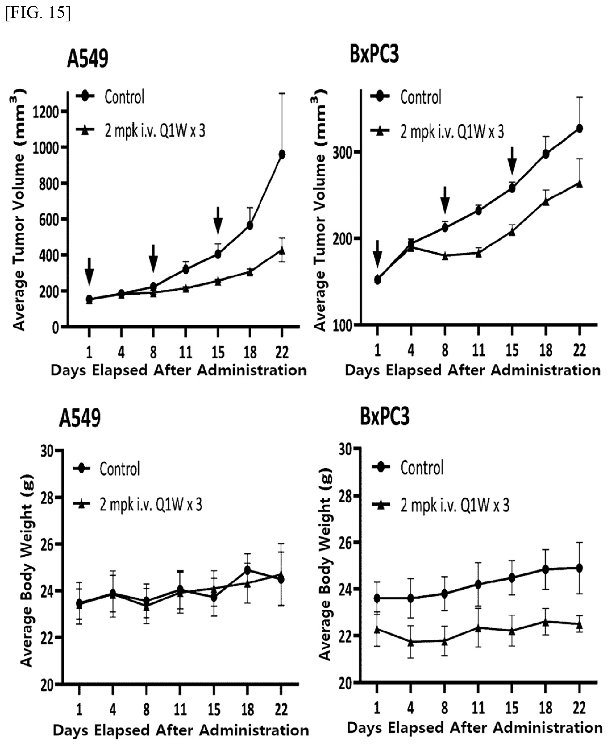

FIG. 15 is a graph showing the results of confirming the tumor growth inhibition effect and body weight change by treatment with mRNA encoding a fusion protein including S14 and E3 ubiquitin ligase fragments, which is one specific example of the present invention, in a tumor mouse model transplanted with human cancer cell lines (A549 cells, BxPC cells).

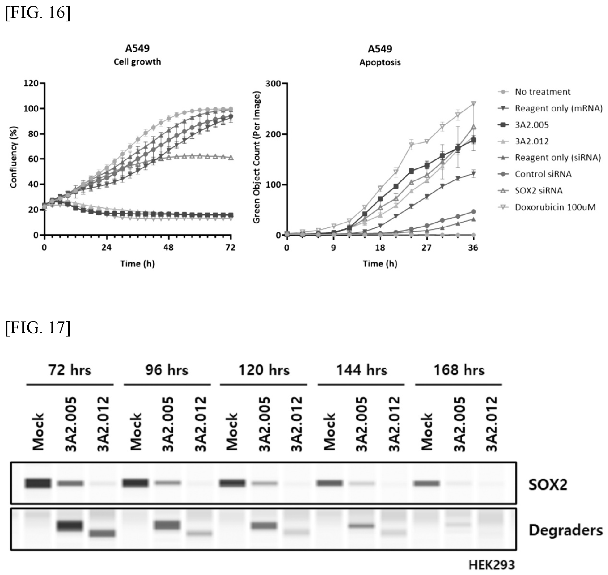

FIG. 16 is a graph showing the results of confirming the cell growth inhibition effect and the apoptosis induction effect by treatment of mRNA encoding a fusion protein including 3A2 and E3 ubiquitin ligase fragments, which is one specific example of the present invention, in a human non-small cell lung cancer cell line (A549 cells).
FIG. 17 is a drawing showing the results of confirming the expression of a fusion protein (degraders) and changes in SOX2 protein expression over time after treating a human kidney cell line (HEK293 cells) with mRNA encoding a fusion protein including 3A2 and E3 ubiquitin ligase fragments, which is one specific example of the present invention.

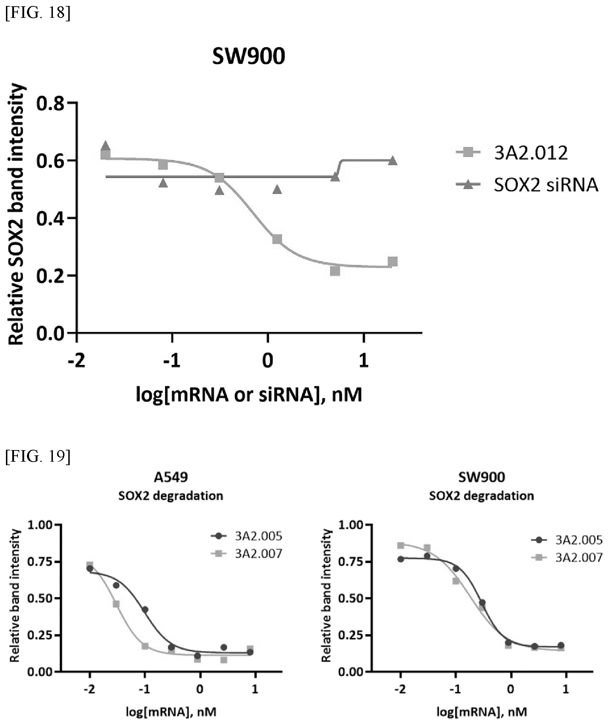

FIG. 18 is a graph showing the results of confirming the change in expression of SOX2 protein according to treatment with different concentrations of mRNA or SOX2 siRNA encoding a fusion protein including 3A2 and E3 ubiquitin ligase fragments, which is one specific example of the present invention, in a human non-small cell lung cancer cell line (SW900 cells).
FIG. 19 is a graph showing the results of confirming the change in expression of SOX2 by treatment with different concentrations of mRNA encoding a fusion protein including 3A2 and E3 ubiquitin ligase fragments, which is one specific example of the present invention, in human non-small cell lung cancer cell lines (A549 cells, SW900 cells).

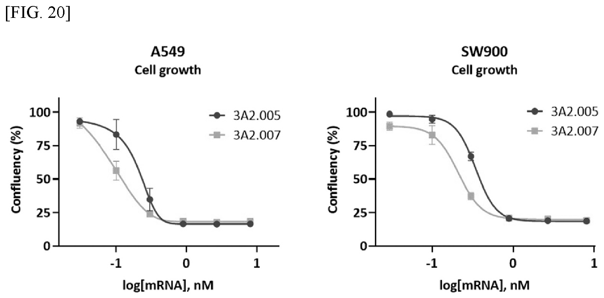

FIG. 20 is a graph showing the results of confirming the cell growth inhibition effect by treatment with different concentrations of mRNA encoding a fusion protein including 3A2 and E3 ubiquitin ligase fragments, which is one specific example of the present invention, in human non-small cell lung cancer cell lines (A549 cells, SW900 cells).

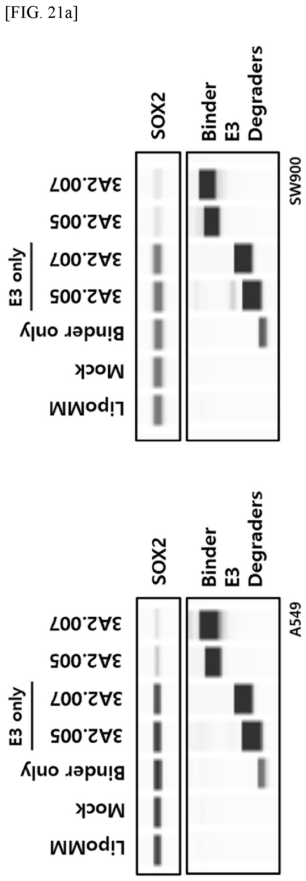

FIG. 21a is a drawing showing the results of comparing the expression of SOX2 protein by treatment with mRNA encoding a 3A2 single domain antibody (binder only), E3 ubiquitin ligase fragment (E3 only) or a fusion protein including 3A2 and E3 ubiquitin ligase fragments, which is one specific example of the present invention, in human non-small cell lung cancer cell lines (A549 cells, SW900 cells). LipoMM: Lipofectamine-MessengerMax reagent only.

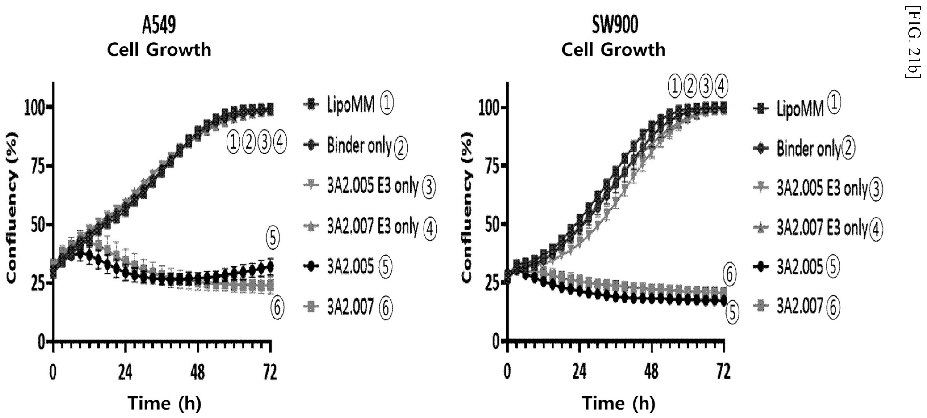

FIG. 21b is a graph showing the results of confirming the cell growth inhibition effect by treatment of mRNA encoding a 3A2 single domain antibody (binder only), an E3 ubiquitin ligase fragment (E3 only) or a fusion protein comprising 3A2 and E3 ubiquitin ligase fragments, which is one specific example of the present invention, in human non-small cell lung cancer cell lines (A549 cells, SW900 cells). LipoMM: Treatment with Lipofectamine-MessengerMax reagent only.

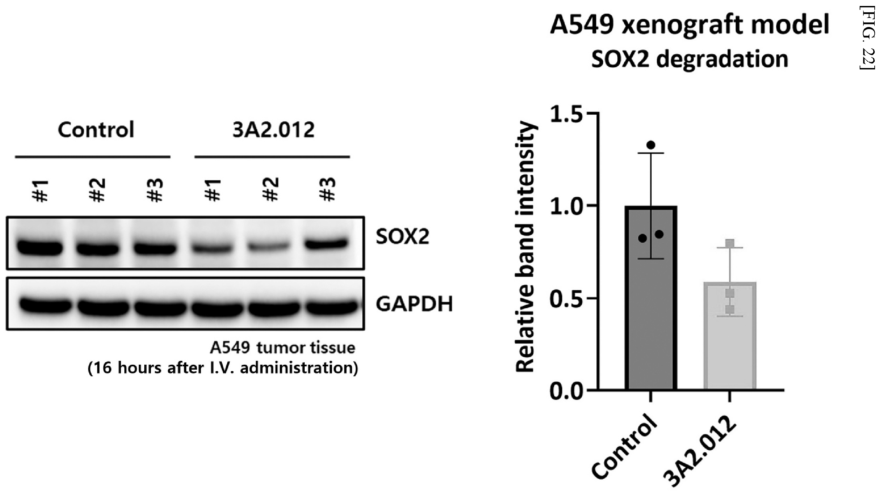

FIG. 22 is a drawing showing the results of confirming the change in the expression of SOX2 protein in tumor tissue by treatment with mRNA encoding a fusion protein including 3A2 and E3 ubiquitin ligase fragments, which is one specific example of the present invention, in a tumor mouse model transplanted with a human cancer cell line (A549 cells), and a graph showing the quantification of the results.

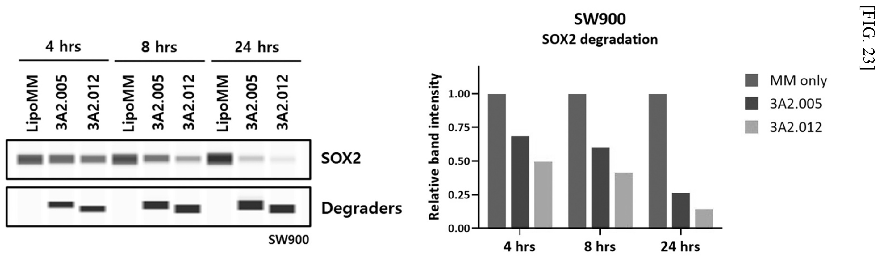

FIG. 23 is a drawing showing the results of confirming the change in the expression of the fusion protein (degrader) and SOX2 protein over time after treatment of mRNA encoding the fusion protein including 3A2 and E3 ubiquitin ligase fragments, which is one specific example of the present invention, in a human non-small cell lung cancer cell line (SW900 cells), and a graph showing the results quantified. MM only or LipoMM: Lipofectamine-MessengerMax reagent only.

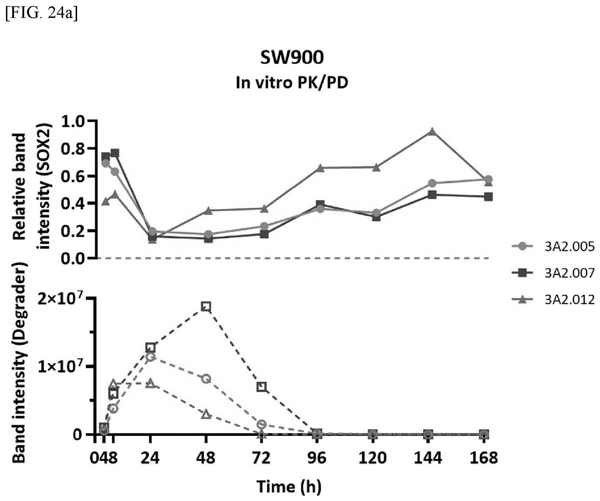

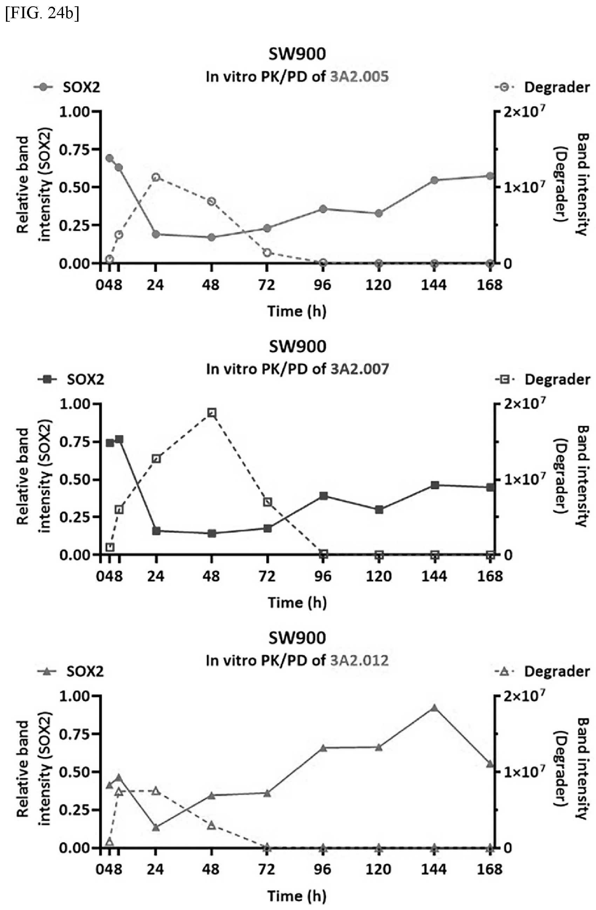

FIGS. 24a and 24b are graphs showing the expression of the fusion protein (degrader) and SOX2 protein over time after treatment of mRNA encoding a fusion protein including 3A2 and E3 ubiquitin ligase fragments, which are one specific example of the present invention, in a human non-small cell lung cancer cell line (SW900 cells).

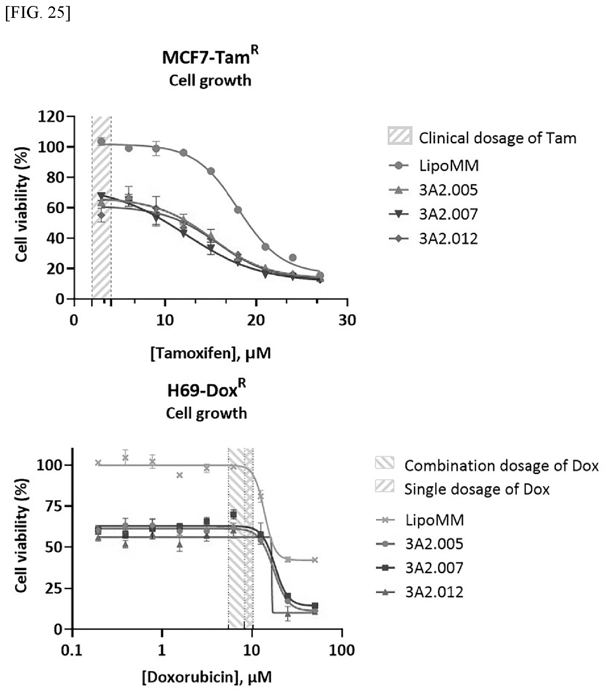

FIG. 25 is a graph showing the results of confirming the cell growth inhibition effect by combined use of mRNA encoding a fusion protein including 3A2 and E3 ubiquitin ligase fragments, which is one specific example of the present invention, and anticancer drugs (tamoxifen or doxorubicin) in each anticancer drug-resistant cancer cell line (MCF7-Tam R cells, H69-Dox R cells). LipoMM: Lipofectamine-MessengerMax reagent only.

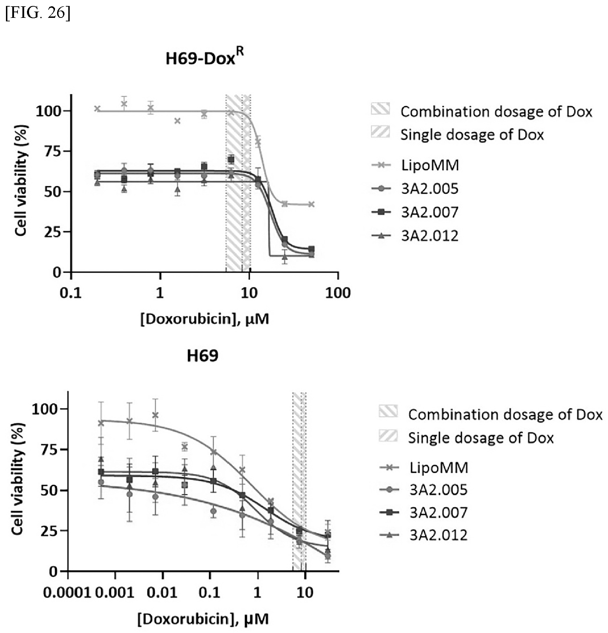

FIG. 26 is a graph showing the results of confirming the cell growth inhibition effect by combined use of mRNA encoding a fusion protein including 3A2 and E3 ubiquitin ligase fragments, which is one specific example of the present invention, and doxorubicin in doxorubicin-resistant cancer cell lines (H69-Dox R cells) or non-resistant cancer cells (H69 cells). LipoMM: Lipofectamine-MessengerMax reagent only.

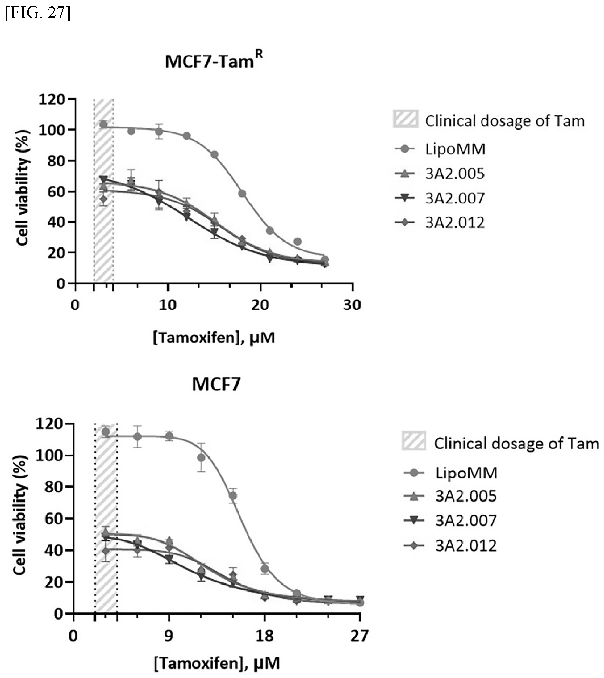

FIG. 27 is a graph showing the results of confirming the cell growth inhibition effect by combined use of mRNA encoding a fusion protein including 3A2 and E3 ubiquitin ligase fragments, which is one specific example of the present invention, and tamoxifen in tamoxifen-resistant cancer cell lines (MCF7-Tam R cells) or non-resistant cancer cells (MCF7 cells). LipoMM: Lipofectamine-MessengerMax reagent only.

### BEST MODE

### Anti-SOX2 single domain antibody or antigen-binding fragment thereof

One aspect of the present invention provides an anti-SOX2 single domain antibody or antigen-binding fragment thereof, including a heavy chain variable region including HCDR1 including any one amino acid sequence selected from the group consisting of SEQ ID NO: 56, SEQ ID NO: 63, SEQ ID NO: 69, SEQ ID NO: 73, SEQ ID NO: 78, SEQ ID NO: 82, SEQ ID NO: 87 and SEQ ID NO: 94; HCDR2 including any one amino acid sequence selected from the group consisting of SEQ ID NO: 58, SEQ ID NO: 65, SEQ ID NO: 71, SEQ ID NO: 72, SEQ ID NO: 74, SEQ ID NO: 75, SEQ ID NO: 76, SEQ ID NO: 79, SEQ ID NO: 84 and SEQ ID NO: 89; and HCDR3 including any one amino acid sequence selected from the group consisting of SEQ ID NO: 60, SEQ ID NO: 67, SEQ ID NO: 81, SEQ ID NO: 85, SEQ ID NO: 91 and SEQ ID NO: 96.

The term "SOX2 (SRY-box transcription factor 2)" used in the present specification is known as a transcription factor that is essential for maintaining the self-renewal or pluripotency of undifferentiated embryonic stem cells. In addition, SOX2 is overexpressed in various types of human cancers, and it has been reported that the overexpression of SOX2 is correlated with a decreased survival rate of cancer patients. SOX2 promotes the proliferation, survival, invasion/metastasis, cancer stemness and drug resistance development of cancer cells. Therefore, SOX2 has recently been attracting attention as a target for cancer treatment.

In the present invention, the SOX2 may include, without limitation, any mammalian SOX2, but preferably refers to human SOX2 or ape SOX2. In addition, the SOX2 protein in the present invention includes, but is not limited to, a natural type, a fragment or a mutant thereof. The "wild type" includes all proteins found in nature or nucleic acids encoding the same, and may be described interchangeably with the wild type. The natural type SOX2 protein generally refers to a polypeptide including the amino acid sequence of the natural type SOX2 protein, and the amino acid sequence of the natural type SOX2 protein generally refers to an amino acid sequence found in naturally occurring SOX2. Information on the SOX2 can be obtained from known databases such as GenBank of the National Institutes of Health in the United States. The SOX2 protein in the present invention may include an amino acid sequence of SEQ ID NO: 1 or SEQ ID NO: 2, but is not limited thereto.

The fragment refers to a fragment in which part of the N-terminus and/or the C-terminus of a natural type SOX2 protein is truncated. Specifically, the SOX2 fragment may be one in which 110, 111, 113, 114, 115, 116, 117, 118, 119, 120, 121, 122, 123, 124, 125, 126, 127, 128, 129, 130, 131, 132, 133 or 134 amino acids are truncated consecutively from the 1^{st} amino acid of the N-terminus of a protein having an amino acid sequence of SEQ ID NO: 1. As a specific example, the SOX2 fragment may include an amino acid sequence of SEQ ID NO: 4.

As used herein, the term "anti-SOX2 single domain antibody" refers to an antibody molecule that specifically binds to SOX2 having a single variable region.

The "single domain antibody (sdAb)" generally refers to an antibody having antigen binding activity that includes only one heavy chain variable region (VH). Mainly, sdAb derived from a heavy chain is used, but it has been reported that a single variable region fragment derived from a light chain also specifically binds to an antigen. In the present invention, the single domain antibody may be an antibody that does not include a light chain and includes a single chain FR1-VHHCDR1-FR2-VHHCDR2-FR3-VHHCDR3-FR4 in the order from the N terminus to the C terminus. The single domain antibody is used interchangeably with a heavy chain antibody, a nanobody VHH, a nanobody or a heavy chain only antibody (HCAb), and although HCAb does not have a light chain, it has a certain antigen binding mechanism part. The variable region (VHH region) of a heavy chain antibody represents the smallest known antigen binding unit generated by an adaptive immune response. The antibodies may include antigen-binding fragments of antibody molecules as well as complete antibody forms.

As used herein, the term "antigen-binding fragment" refers to one or more fragments of an intact antibody that retain the capability to specifically bind to a given antigen, that is, SOX2. The antibody fragments in the present invention include, but are not limited to, single-chain antibodies, bispecific antibodies, trispecific antibodies, multispecific antibodies such as diabodies, triabodies and tetrabodies, Fab fragments, F(ab')₂ fragments, Fd, scFv, domain antibodies, minibodies, sterol regulatory binding protein cleavage activating protein (scap), chelating recombinant antibodies, bibodies, intrabodies, nanobodies, small modular immunopharmaceuticals (SMIPs), binding domain immunoglobulin fusion proteins, camelized antibodies, VHH-containing antibodies, IgD antibodies, IgE antibodies, IgM antibodies, IgG1 antibodies, IgG2 antibodies, IgG3 antibodies, IgG4 antibodies, antibody constant region derivatives, and synthetic antibodies based on protein scaffolds having binding capability to SOX2.

It will be apparent to those skilled in the art that any fragment of an antibody according to the present invention will exhibit the same properties as the antibody according to the present invention, as long as the binding function to SOX2 is maintained.

As used herein, the term "heavy chain (HC)" refers to a full-length heavy chain and fragments thereof, which include a variable region domain VH including an amino acid sequence having sufficient variable region sequence to confer specificity to an antigen and three constant region domains CH1, CH2 and CH3.

The term "variable (V)" as used herein indicates that a specific part of the variable region differs significantly in sequence among antibodies. The variable region mediates antigen binding and determines the specificity of a particular antibody for its particular antigen. The variability is concentrated in three segments, called hypervariable regions (HVRs), that is, CDRs, in all heavy-chain variable regions. The more highly conserved parts of the variable region are called the framework (FR) regions. The heavy-chain variable region has the following structures, from N-terminus to C-terminus: FR1, CDR1, FR2, CDR2, FR3, CDR3 and FR4.

As used herein, the term " complementarity determining region (CDR)" refers to the amino acid sequence of the hypervariable region (HVR) of an immunoglobulin heavy chain. Each heavy chain (HCDR1, HCDR2 and HCDR3) includes three CDRs. CDRs provide key contact residues for binding of an antibody to an antigen or epitope.

In the present invention, the anti-SOX2 single domain antibody or antigen-binding fragment thereof may include a heavy chain variable region including: HCDR1 including any one amino acid sequence selected from the group consisting of SEQ ID NO: 56, SEQ ID NO: 63, SEQ ID NO: 69, SEQ ID NO: 73, SEQ ID NO: 78, SEQ ID NO: 82, SEQ ID NO: 87 and SEQ ID NO: 94; HCDR2 including any one amino acid sequence selected from the group consisting of SEQ ID NO: 58, SEQ ID NO: 65, SEQ ID NO: 71, SEQ ID NO: 72, SEQ ID NO: 74, SEQ ID NO: 75, SEQ ID NO: 76, SEQ ID NO: 79, SEQ ID NO: 84 and SEQ ID NO: 89; and HCDR3 including any one amino acid sequence selected from the group consisting of SEQ ID NO: 60, SEQ ID NO: 67, SEQ ID NO: 81, SEQ ID NO: 85, SEQ ID NO: 91 and SEQ ID NO: 96.

In one specific embodiment, the anti-SOX2 single domain antibody or antigen-binding fragment thereof may include a heavy chain variable region including HCDR1 including an amino acid sequence of SEQ ID NO: 56, HCDR2 including an amino acid sequence of SEQ ID NO: 58, and HCDR3 including an amino acid sequence of SEQ ID NO: 60. The heavy chain variable region may include an amino acid sequence of SEQ ID NO: 5.

In one specific embodiment, the anti-SOX2 single domain antibody or antigen-binding fragment thereof may include a heavy chain variable region including HCDR1 including an amino acid sequence of SEQ ID NO: 63, HCDR2 including an amino acid sequence of SEQ ID NO: 65; and HCDR3 including an amino acid sequence of SEQ ID NO: 67. The heavy chain variable region may include an amino acid sequence of SEQ ID NO: 6.

In one specific embodiment, the anti-SOX2 single domain antibody or antigen-binding fragment thereof may include a heavy chain variable region including HCDR1 including an amino acid sequence of SEQ ID NO: 69, HCDR2 including an amino acid sequence of SEQ ID NO: 71; and HCDR3 including an amino acid sequence of SEQ ID NO: 67. The heavy chain variable region may include an amino acid sequence of SEQ ID NO: 7.

In one specific embodiment, the anti-SOX2 single domain antibody or antigen-binding fragment thereof may include HCDR1 including an amino acid sequence of SEQ ID NO: 63, HCDR2 including an amino acid sequence of SEQ ID NO: 72; and HCDR3 including an amino acid sequence of SEQ ID NO: 67. The heavy chain variable region may include an amino acid sequence of SEQ ID NO: 8.

In one specific embodiment, the anti-SOX2 single domain antibody or antigen-binding fragment thereof may include a heavy chain variable region including HCDR1 including an amino acid sequence of SEQ ID NO: 73, HCDR2 including an amino acid sequence of SEQ ID NO: 74; and HCDR3 including an amino acid sequence of SEQ ID NO: 67. The heavy chain variable region may include an amino acid sequence of SEQ ID NO: 9.

In one specific embodiment, the anti-SOX2 single domain antibody or antigen-binding fragment thereof may include a heavy chain variable region including HCDR1 including an amino acid sequence of SEQ ID NO: 63, HCDR2 including an amino acid sequence of SEQ ID NO: 75; and HCDR3 including an amino acid sequence of SEQ ID NO: 67. The heavy chain variable region may include an amino acid sequence of SEQ ID NO: 10 or SEQ ID NO: 11.

In one specific embodiment, the anti-SOX2 single domain antibody or antigen-binding fragment thereof may include a heavy chain variable region including HCDR1 including an amino acid sequence of SEQ ID NO: 63, HCDR2 including an amino acid sequence of SEQ ID NO: 76; and HCDR3 including an amino acid sequence of SEQ ID NO: 67. The heavy chain variable region may include an amino acid sequence of SEQ ID NO: 12.

In one specific embodiment, the anti-SOX2 single domain antibody or antigen-binding fragment thereof may include a heavy chain variable region including HCDR1 including an amino acid sequence of SEQ ID NO: 82, HCDR2 including an amino acid sequence of SEQ ID NO: 84; and HCDR3 including an amino acid sequence of SEQ ID NO: 85. The heavy chain variable region may include an amino acid sequence of SEQ ID NO: 13.

In one specific embodiment, the anti-SOX2 single domain antibody or antigen-binding fragment thereof may include a heavy chain variable region including HCDR1 including an amino acid sequence of SEQ ID NO: 78, HCDR2 including an amino acid sequence of SEQ ID NO: 79; and HCDR3 including an amino acid sequence of SEQ ID NO: 81. The heavy chain variable region may include an amino acid sequence of SEQ ID NO: 14.

In one specific embodiment, the anti-SOX2 single domain antibody or antigen-binding fragment thereof may include a heavy chain variable region including HCDR1 including an amino acid sequence of SEQ ID NO: 87, HCDR2 including an amino acid sequence of SEQ ID NO: 89; and HCDR3 including an amino acid sequence of SEQ ID NO: 91. The heavy chain variable region may include an amino acid sequence of SEQ ID NO: 15.

In one specific embodiment, the anti-SOX2 single domain antibody or antigen-binding fragment thereof may include a heavy chain variable region including HCDR1 including an amino acid sequence of SEQ ID NO: 94, HCDR2 including an amino acid sequence of SEQ ID NO: 89; and HCDR3 including an amino acid sequence of SEQ ID NO: 91. The heavy chain variable region may include an amino acid sequence of SEQ ID NO: 16.

In one specific embodiment, the anti-SOX2 single domain antibody or antigen-binding fragment thereof may include a heavy chain variable region including HCDR1 including an amino acid sequence of SEQ ID NO: 94, HCDR2 including an amino acid sequence of SEQ ID NO: 89; and HCDR3 including an amino acid sequence of SEQ ID NO: 96. The heavy chain variable region may include an amino acid sequence of SEQ ID NO: 17.

In addition, the heavy chain variable region of the antibody may include an amino acid sequence having about 90%, about 91%, about 92%, about 93%, about 94%, about 95%, about 96%, about 97%, about 98%, about 99% identity or about 100% identity to an amino acid sequence of SEQ ID NO: 5 to SEQ ID NO: 17.

### Polynucleotide encoding anti-SOX2 single domain antibody or antigen-binding fragment thereof

Another aspect of the present invention provides a polynucleotide encoding an anti-SOX2 single domain antibody or an antigen-binding fragment thereof. The polynucleotide may include any one base sequence selected from the group consisting of SEQ ID NO: 101 to SEQ ID NO: 113.

The anti-SOX2 single domain antibody and antigen binding fragment are the same as those described above.

As used herein, the term "polynucleotide", also referred to as "nucleic acid", refers to a polymer of nucleotides of any length. Specifically, the polynucleotide may be DNA or RNA.

The polynucleotide may be mutated by substitution, deletion, insertion or a combination thereof of one or more bases, as long as it encodes the same polypeptide. When the polynucleotide sequence is prepared by chemical synthesis, synthetic methods well known in the art may be used, for example, the method described in the literature (Engels and Uhlmann, Angew Chem IntEd Engl., 37:73-127, 1988), and examples thereof include triester, phosphite, phosphoramidite and H-phosphate methods, PCR and other autoprimer methods, and oligonucleotide synthesis on solid supports.

Additionally, in the present invention, the polynucleotide may comprise or consist of a base sequence having about 70%, about 75%, about 80%, about 85%, about 86%, about 87%, about 88%, about 89%, about 90%, about 91%, about 92%, about 93%, at least about 94%, about 95%, about 96%, about 97%, about 98%, about 99% or about 100% identity with the base sequences of SEQ ID NO: 101 to SEQ ID NO: 113.

The polynucleotide may additionally include a signal sequence or a leader sequence.

### Vector loaded with polynucleotide encoding anti-SOX2 single domain antibody or antigen-binding fragment thereof

Still another aspect of the present invention provides an expression vector loaded with a polynucleotide encoding an anti-SOX2 single domain antibody or an antigen-binding fragment thereof. The anti-SOX2 single domain antibody, antigen-binding fragment and polynucleotide are the same as described above.

As used herein, the term "vector" means a material for carrying or expressing a nucleic acid sequence including a nucleic acid sequence encoding an anti-SOX2 single domain antibody variant described herein.

Specifically, the vector includes linear nucleic acids, plasmids, phagemids, cosmids, RNA vectors, viral vectors and analogs thereof. Examples of viral vectors include, but are not limited to, retroviruses, adenoviruses and adeno-associated viruses. In addition, the plasmid may include a selection marker, such as an antibiotic resistance gene, and the host cell maintaining the plasmid may be cultured under selective conditions.

More specifically, the vector may be a plasmid DNA, a phage DNA or the like, and may be a commercially developed plasmid (e.g., pUC18, pBAD, pIDTSAMRT-AMP, etc.), an E. coli-derived plasmid (e.g., pYG601BR322, pBR325, pUC118, pUC119, etc.), a Bacillus subtilis-derived plasmid (e.g., pUB110, pTP5, etc.), a yeast-derived plasmid (e.g., YEp13, YEp24, YCp50, etc.), a phage DNA (e.g., Charon4A, Charon21A, EMBL3, EMBL4, λgt10, λgt11, λZAP, etc.), an animal virus vector (e.g., Retrovirus, Adenovirus, Vaccinia virus, etc.) or an insect virus vector (Baculovirus, etc.). Since the vector shows different protein expression levels and formulas depending on the host cell, it is desirable to select and use the host cell that is most suitable for the purpose.

In addition, the vector of the present invention may be fused with other sequences to facilitate the purification of antibodies expressed therefrom. The sequences to be fused include, for example, Flag (IBI, USA), glutathione S-transferase (Pharmacia, USA), maltose binding protein (NEB, USA), 6×His (Qiagen, USA), Halo or Myc.

In addition, since the protein expressed by the vector of the present invention is an antibody, the expressed antibody may be easily purified through a Protein A column or the like without an additional sequence for purification.

### Transformed cell expressing anti-SOX2 single domain antibody or antigen-binding fragment thereof

Still another aspect of the present invention provides a transformed cell into which an expression vector loaded with a polynucleotide encoding the anti-SOX2 single domain antibody or an antigen-binding fragment thereof is introduced. The anti-SOX2 single domain antibody, the antigen-binding fragment, the polynucleotide and the expression vector are the same as described above.

The term "transformed cell" as used herein refers to prokaryotic cells and eukaryotic cells into which a recombinant expression vector can be introduced. The transformed cell may be produced by introducing the vector into a host cell and transforming the same. In addition, the polynucleotide included in the vector may be expressed to produce the anti-SOX2 single domain antibody of the present invention or an antigen-binding fragment thereof.

The transformation may be performed by various methods. It is not particularly limited thereto, as long as it can produce the anti-SOX2 single domain antibody of the present invention or an antigen-binding fragment thereof. Specifically, the transformation method may be a CaCl₂ precipitation method, a Hanahan method with increased efficiency by using a reducing substance called dimethyl sulfoxide (DMSO) in the CaCl₂ precipitation method, electroporation, a calcium phosphate precipitation method, a protoplast fusion method, a stirring method using a silicon carbide fiber, an *Agrobacterium*-mediated transformation method, a transformation method using PEG, dextran sulfate, lipofectamine and a drying/inhibition-mediated transformation method. In addition, a target substance may be delivered into a cell by using a virus particle by infection. In addition, a vector may be introduced into a host cell by gene bombardment and the like.

In addition, the host cell used for producing the transformed cell is also not particularly limited thereto, as long as it can produce the antibody of the present invention. Specifically, the host cell may include, but is not limited to, a prokaryotic cell, a eukaryotic cell, a cell of a mammal, a plant, an insect, a fungus or cellular origin. An example of the prokaryotic cell may be *Escherichia coli*. In addition, an example of the eukaryotic cell may be yeast. In addition, as the mammalian cell, CHO cells, F2N cells, COS cells, BHK cells, Bowes melanoma cells, HeLa cells, 911 cells, AT1080 cells, A549 cells, SP2/0 cells, human lymphoblastoid, NSO cells, HT-1080 cells, PERC.6 cells, HEK293 cells or HEK293T cells may be used, but is not limited thereto, and any cell known to those skilled in the art that can be used as a mammalian host cell may be used.

Additionally, in order to optimize the properties of the antibody as a therapeutic agent or for other purposes, the glycosylation-related genes of the host cell may be manipulated by methods known to those skilled in the art to adjust the sugar chain pattern of the antibody (e.g., sialic acid, fucosylation, glycosylation).

### Method for producing anti-SOX2 single domain antibody or antigen-binding fragment thereof

Still another aspect of the present invention provides a method for producing an anti-SOX2 single domain antibody or antigen-binding fragment thereof, including culturing the transformed cell.

The anti-SOX2 single domain antibody, antigen-binding fragment and transformed cells are the same as described above.

Specifically, the method for producing the antibody or fragment thereof may include culturing a transformed cell to produce an antibody and obtaining the produced antibody from the culture medium.

The method for culturing the transformed cell may be performed by using a method widely known in the art. Specifically, the culturing is not particularly limited thereto as long as it can express and produce the anti-SOX2 single domain antibody of the present invention or an antigen-binding fragment thereof. Specifically, the culturing may be continuously cultured in a batch process or a fed batch or repeated fed batch process.

In addition, the step of obtaining the antibody and fragment thereof specific to SOX2 from the culture medium may be performed by a method known in the art. Specifically, the obtaining method is not particularly limited thereto, as long as the antibody of the present invention or the fragment thereof produced can be obtained. Preferably, the obtaining method may be a method such as centrifugation, filtration, extraction, spraying, drying, evaporation, precipitation, crystallization, electrophoresis, differential dissolution (e.g., ammonium sulfate precipitation) or chromatography (e.g., ion exchange, affinity, hydrophobicity, and size exclusion).

### Fusion protein including anti-SOX2 single domain antibody or antigen-binding fragment thereof and fragment of E3 ubiquitin ligase or variant thereof

Still another aspect of the present invention provides a fusion protein including the anti-SOX2 single domain antibody or an antigen-binding fragment thereof; and a fragment of E3 ubiquitin ligase or a variant thereof.

The anti-SOX2 single domain antibodies and antigen binding fragments are the same as described above.

The term "ubiquitin (UB)" used in the present specification is a protein composed of 76 amino acids, which exists in almost all eukaryotic cells. Ubiquitin has the property of covalently bonding to other proteins through an ATP-dependent reaction, which is called ubiquitination.

The ubiquitin is covalently bonded to a target protein (hereinafter, referred to as a substrate protein) through 1) activation performed by ubiquitin-activating enzyme (E1), ubiquitin-conjugating enzyme (E2) and ubiquitin ligase (E3), 2) conjugation, and 3) ligation. The target protein ubiquitinated (poly-Ub) as described above is degraded by the proteasome.

The term "E3 ubiquitin ligase" as used herein refers to an enzyme in the final step of ubiquitination that attaches ubiquitin to a substrate. E3 ligase binds to a target protein and E2-Ub, thereby inducing ubiquitination (poly-Ub) of the target protein by positioning the target protein close to E2-Ub. In the present invention, the E3 ubiquitin ligase may be used interchangeably as E3 ligase, E3 ubiquitin ligase, E3 ubiquitin conjugation enzyme or E3.

As used herein, the term "fragment" may refer to a specific substrate recognition domain, a ubiquitin transfer domain, or two domains thereof within an E3 ubiquitin ligase complex.

Table 1 below shows the subunit types and examples of E3 ubiquitin ligase complexes according to CRL type.

**[Table 1]**

| Cullin protein | Adapter | Subunit | Example |
|---|---|---|---|
| Cul1 | Skp1 | F-box protein | Skp2, βFbw, Fbxo, Fbxl |
| Cul2 | EloBC | VHL-box protein | VHL |
| Cul3 | BTB | BTB domain protein | KLHL, SPOP |
| Cul4A | DDB1 | DCAF/H-box protein | DCAF, DDB2, CSA, CDT2, CRBN |
| Cul4B | DDB1 | DCAF/H-box protein | DCAF, DDB2 |
| Cul5 | EloBC | SOCS-box protein | SOCS1~SOCS7 |
| Cul7 | Skp1 | F-box protein | Fbxw8 |

In the present invention, the fragment of the E3 ubiquitin ligase or a variant thereof may be any one selected from the group consisting of βTrCP (beta-transducin repeat-containing protein), SKP2 (S-phase kinase-associated protein 2), VHL (von Hippel-Lindau), SPOP (speckle-type BTB-POZ), SOCS2 (suppressor of cytokine signaling 2), CHIP (carboxy terminus of Hsp70-binding protein), DDB2 (damage DNA binding protein 2), CRBN (cereblon), ASB1 (ankyrin repeat and SOCS box protein 1), TRIM21 (tripartite motif-containing protein 21), RNF4 (RING finger protein), UBE2D1 (ubiquitin conjugating enzyme E2 D1), UBE2D4 and ZNFR1 (zinc and ring finger 1) and specific domains thereof.

In one specific example, the mutant of βTrCP may be a βTrCP₂₋₂₆₃ (amino acids 2-263, SEQ ID NO: 206) fragment or a βTrCP₁₉₀₋₂₂₈ (amino acids 190-228, SEQ ID NO: 18) fragment of the full-length (Uniprot ID: Q9Y297) of βTrCP. In one specific example, the variant of SKP2 may be a SKP2₂₋₁₇₆ (amino acids 2-176, SEQ ID NO: 19) fragment of the full-length SKP2 (Uniprot ID: Q13309). In one specific example, the variant of VHL may be a VHL₁₅₂₋₂₁₃ (amino acids 152-213, SEQ ID NO: 20) fragment of the full-length VHL (Uniprot ID: P40337). In one specific embodiment, the variant of SPOP may be a fragment of SPOP₁₆₇₋₃₇₄ (amino acids 167-374, SEQ ID NO: 21) of the full-length SPOP (Uniprot ID: O43791). In one specific embodiment, the variant of SOCS2 may be a fragment of SOCS2₁₄₃₋₁₉₈ (amino acids 143-198, SEQ ID NO: 22) of the full-length SOCS2 (Uniprot ID: 014508). In one specific embodiment, the variant of CHIP may be a fragment of CHIP₁₂₈₋₃₀₃ (amino acids 128-303, SEQ ID NO: 23) of the full-length CHIP (Uniprot ID: Q9UNE). In one specific embodiment, the variant of DDB2 may be a fragment of DDB2₂₋₁₁₄ (amino acids 2-114, SEQ ID NO: 24) of the full-length DDB2 (Uniprot ID: Q92466). In one specific embodiment, the variant of CRBN may be a fragment of CRBN₂₋₃₂₀ (amino acids 2-320, SEQ ID NO: 25) of the full-length CRBN (Uniprot ID: Q96SW2). In one specific embodiment, the variant of ASB1 may be a fragment of ASB1₂₆₆₋₃₃₅ (amino acids 266-335, SEQ ID NO: 26) of the full-length ASB1 (Uniprot ID: Q9Y576). In one specific example, the variant of TRIM21 may be a fragment of TRIM21₂₋₈₅ (amino acids 2-85, SEQ ID NO: 27) of the full-length TRIM21 (Uniprot ID: P19474). In one specific example, the variant of RNF4 may be a fragment of RNF4₇₁₋₁₉₀ (amino acids 71-190, SEQ ID NO: 28) of the full-length RNF4 (Uniprot ID: P78317). In one specific example, the variant of UBE2D1 may be a fragment of UBE2D1₂₋₁₄₇ (amino acids 2-147, SEQ ID NO: 29) of the full-length UBE2D1 (Uniprot ID: P51668). In one specific example, the variant of UBE2D4 may be a fragment of UBE2D4₂₋₁₄₇ (amino acids 2-147, SEQ ID NO: 30) of the full-length UBE2D4 (Uniprot ID: Q9Y2X8). In one specific example, the variant of ZNFR1 may be a fragment of ZNFR1₁₃₈₋₂₂₇ (amino acids 138-227, SEQ ID NO: 31) of the full-length ZNFR1 (Uniprot ID: Q8ND2).

In one specific embodiment, the fragment or variant thereof of the E3 ubiquitin ligase may include any one amino acid sequence selected from the group consisting of SEQ ID NO: 18 to SEQ ID NO: 31 and SEQ ID NO: 206.

The variant of the fragment of the E3 ubiquitin ligase refers to a form in which some of the amino acids of the fragment or a fragment thereof are substituted. That is, the variant of the fragment refers to a peptide having a sequence different from that of the natural fragment or a fragment thereof and having the function of binding to an adaptor protein.

In one specific example, the variant of the fragment may have 5 to 18 amino acids substituted in the natural type βTrCP fragment.

More specifically, in the βTrCP variant, any one amino acid selected from the group consisting of the 8th, 10th, 11th, 16th, 18th, 28th, 33rd, 35th, 38th, 47th, 91st, 95th, 98th, 102nd, 105th, 110th, 114th, 115th, 120th, 122nd, 124th, 125th, 127th, 132nd, 144th, 146th, 148th, 159th, 170th, 172nd, 173rd, 183rd, 185th, 192nd, 197th, 198th, 202nd, 203rd, 204th, 225th, 234th, 236th, 240th, 241st, 242nd, 250th, 251st, 260th and combinations thereof may be substituted.

More specifically, in the variant, any one amino acid selected from the group consisting of L8, E10, K11, M16, S18, E28, I33, P35, N38, C47, S91, E95, K98, V102, F105, E110, V114, E115, L120, S122, M124, C125, Y127, I132, F144, T146, L148, N159, C170, A172, E173, T183, D185, L192, V197, R198, L202, W203, R204, N225, A234, Y236, I240, Q241, D242, W250, R251, I260 and combinations thereof may be substituted for another amino acid in the amino acid sequence of SEQ ID NO: 206.

In this case, the "amino acid" introduced by the substitution may be any one selected from the group consisting of glycine, alanine, valine, leucine, isoleucine, serine, threonine, cysteine, methionine, aspartic acid, glutamic acid, asparagine, glutamine, lysine, arginine, phenylalanine, tyrosine, tryptophan, histidine and proline.

More specifically, the variant may be substituted with any one amino acid selected from the group consisting of L8P, E10V, K11E, M16L, S18T, E28G, I33T, P35A, N38D, C47S, S91T, E95G, K98R, V102A, F105L, E110G, V114A, E115G, L120P, S122P, M124V, C125R, Y127C, I132T, F144S, T146P, L148P, N159K, C170R, A172V, E173V, T183S, D185G, D185E, L192P, V197A, R198G, L202Q, L202R, W203R, R204G, N225Y, A234V, Y236C, I240M, Q241R, D242E, W250R, R251G, I260S and combinations thereof in the amino acid sequence of SEQ ID NO: 206. In this case, when D185 is substituted, it may be substituted with D185G or D185E. In addition, L202 may be substituted with L202Q or L202R.

The βTrCP variant may comprise or consist of any one amino acid sequence selected from the group consisting of SEQ ID NO: 207 to SEQ ID NO: 214.

In one specific example, the variant of the fragment may have 7 to 14 amino acids substituted in the natural type SKP2 fragment.

More specifically, in the SKP2 fragment variant, any one amino acid selected from the group consisting of the 2nd, 4th, 11th, 12th, 19th, 24th, 29th, 33rd, 44th, 59th, 78th, 98th, 102nd, 107th, 111th, 112th, 113th, 124th, 128th, 130th, 133rd, 135th, 137th, 138th, 139th, 148th, 156th, 161st, 176th and combinations thereof may be substituted in the amino acid sequence of SEQ ID NO: 19.

More specifically, in the variant, any one amino acid selected from the group consisting of H2, K4, D11, L12, S19, W24, T29, L33, E44, L59, D78, D98, D102, G107, C111, L112, C113, C124, Y128, L130, D133, S135, W137, Q138, T139, H148, L156, I161, E176 and combinations thereof may be substituted with another amino acid in the amino acid sequence of SEQ ID NO: 19. In this case, the amino acid introduced for the substitution is the same as described above.

More specifically, the variant may be substituted with an amino acid selected from the group consisting of H2R, K4R, D11G, L12P, S19G, W24R, T29A, L33P, E44K, L59P, D78G, D98V, D102N, G107R, C111R, L112P, L112R, C113R, C124S, Y128C, L130Q, D133E, S135P, W137R, Q138K, T139S, H148R, L156Q, L156R, I161A, I161V, E176G and combinations thereof in the amino acid sequence of SEQ ID NO: 19. In this case, when L112 is substituted, it may be substituted with L112P or L112R. When L156 is substituted, it may be substituted with L156Q or L156R. When I161 is substituted, it may be substituted with I161A or I161V.

The SKP2 variant may comprise or consist of any one amino acid sequence selected from the group consisting of SEQ ID NO: 215 to SEQ ID NO: 221.

In the present invention, the fusion protein may include a linker. In this case, the linker may be a peptide linker. In the present invention, the anti-SOX2 single domain antibody or an antigen-binding fragment thereof of the fusion protein and the fragment of E3 ubiquitin ligase or a variant thereof may be linked via the linker.

### Structure of fusion protein

Specifically, the fusion protein may consist of Structural Formula (I) or (II) below.

N'-TB-(L)n-SR-C' (I)

N'-SR-(L)n-TB-C' (II)

In this case, in Structural Formulas (I) and (II) above,
the N' is an N-terminus of the fusion protein,
the C' is a C-terminus of the fusion protein,
the TB is an anti-SOX2 single domain antibody or an antigen-binding fragment thereof,
the SR is a fragment of E3 ubiquitin ligase or a variant thereof,
the L is a peptide linker, and
the n is 0 or 1.

In this case, the anti-SOX2 single domain antibody, antigen binding fragment and fragment of E3 ubiquitin ligase or variant thereof are the same as described above.

The peptide linker may consist of 1 to 30 consecutive amino acids, or 2 to 20 consecutive amino acids, or 2 to 10 amino acids. In one specific example, the peptide linker may be (GS)n (wherein n is an integer from 1 to 10). Here, n in (GS)n may be 1, 2, 3, 4, 5, 6, 7, 8, 9 or 10. The peptide linker may use, without particular limitation, a sequence linker known in the art such as GS, GGGGS, (GGGGS)₂, (GGGS)₃, ASTKGP, ASTKGPSVFPLAP and the like, which provides structural flexibility without being cleaved by proteolytic enzymes. As a specific example, the peptide linker may include an amino acid sequence of SEQ ID NO: 157 (amino acid sequence: GS) or SEQ ID NO: 238.

In one specific example, the fusion protein may include any one amino acid sequence selected from the group consisting of SEQ ID NO: 164 to SEQ ID NO: 184.

### Polynucleotide encoding fusion protein including anti-SOX2 single domain antibody and fragment of E3 ubiquitin ligase

Still another aspect of the present invention provides a polynucleotide encoding a fusion protein including the anti-SOX2 single domain antibody or antigen-binding fragment thereof; and a fragment of E3 ubiquitin ligase or a variant thereof. Specifically, the polynucleotide encoding the fusion protein may include a base sequence of SEQ ID NO: 185 to SEQ ID NO: 205.

The anti-SOX2 single domain antibody, antigen binding fragment, E3 ubiquitin ligase and fragment are the same as described above.

In addition, the polynucleotide may include a base sequence having at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99% or at least about 100% identity to the base sequence of SEQ ID NO: 185 to SEQ ID NO: 205, respectively.

The polynucleotide may be mutated by substitution, deletion, insertion or a combination thereof of one or more bases. When the nucleotide sequence is prepared by chemical synthesis, synthetic methods well known in the art, for example, the method described in the literature (Engels and Uhlmann, Angew Chem IntEd Engl., 37:73-127, 1988) may be used.

### Vector loaded with polynucleotide encoding a fusion protein

Still another aspect of the present invention provides an expression vector loaded with a polynucleotide encoding a fusion protein including the anti-SOX2 single domain antibody or antigen-binding fragment thereof; and a fragment of E3 ubiquitin ligase or a variant thereof.

The anti-SOX2 single domain antibody, antigen-binding fragment, E3 ubiquitin ligase, fragment, fusion protein and polynucleotide are the same as described above.

In one specific example, the polynucleotide encoding the fusion protein may include a base sequence of SEQ ID NO: 185 to SEQ ID NO: 205.

Specifically, the vector includes linear nucleic acids, plasmids, phagemids, cosmids, RNA vectors, viral vectors and analogs thereof. Examples of viral vectors include, but are not limited to, retroviruses, adenoviruses and adeno-associated viruses. In addition, the plasmid may include a selection marker, such as an antibiotic resistance gene, and the host cell maintaining the plasmid may be cultured under selective conditions.

In addition, the vector of the present invention may be fused with other sequences to facilitate the purification of antibodies expressed therefrom. The sequences to be fused include, for example, Flag (IBI, USA), glutathione S-transferase (Pharmacia, USA), maltose binding protein (NEB, USA), 6×His (Qiagen, USA), Halo or Myc.

### Transformed cell expressing fusion protein

Still another aspect of the present invention provides a transformed cell, in which an expression vector loaded with a polynucleotide encoding a fusion protein including the anti-SOX2 single domain antibody or antigen-binding fragment thereof; and a fragment of E3 ubiquitin ligase or a variant thereof is introduced.

The anti-SOX2 single domain antibody, antigen-binding fragment, E3 ubiquitin ligase, fragment, variant, fusion protein, polynucleotide and expression vector are the same as described above.

The transformed cell may be produced by introducing a vector into a host cell and transforming the same. In addition, the polynucleotide included in the vector may be expressed to produce the fusion protein of the present invention.

The transformation may be performed by various methods, such as CaCl₂ precipitation, Hanahan method, electroporation, calcium phosphate precipitation, protoplast fusion, Agrobacterium-mediated transformation, transformation using PEG and the like, but is not particularly limited thereto, as long as a fusion protein including the anti-SOX2 single domain antibody variant of the present invention and the variant of the E3 ubiquitin ligase fragment can be produced.

In addition, the host cell used for producing the transformed cell is not particularly limited as long as it can produce the anti-SOX2 single domain antibody variant of the present invention.

### Method for producing fusion protein

Still another aspect of the present invention provides a method for producing the fusion protein, including culturing the transformed cell; and obtaining a fusion protein including an anti-SOX2 single domain antibody or an antigen-binding fragment thereof, and a fragment of E3 ubiquitin ligase or a variant thereof from a culture medium of the cell.

The method for culturing the transformed cell may be performed by using a method widely known in the art. Specifically, the culturing may be performed continuously in a batch process or a fed batch or repeated fed batch process.

In addition, the step of recovering the fusion protein from a culture may be performed by a method known in the art. Specifically, the recovery method is not particularly limited thereto, as long as it can recover the produced fusion protein of the present invention. Preferably, the recovery method may be a method such as centrifugation, filtration, extraction, spraying, drying, evaporation, precipitation, crystallization, electrophoresis, differential dissolution (e.g., ammonium sulfate precipitation) or chromatography (e.g., ion exchange, affinity, hydrophobicity, and size exclusion).

### Pharmaceutical composition including fusion protein, polynucleotide encoding same or vector loaded with polynucleotide

Still another aspect of the present invention provides a pharmaceutical composition for preventing or treating cancer, including the fusion protein, a polynucleotide encoding the fusion protein or a vector loaded with the polynucleotide as an active ingredient. In this case, the polynucleotide may be DNA, mRNA, plasmid DNA and the like, and the polynucleotide or the vector including (loaded with) the same may be delivered into a cell by a cell-penetrating functional nano-carrier and exhibit the same target protein degradation efficacy as the fusion protein.

The fusion protein, the polynucleotide encoding the fusion protein and the vector including the polynucleotide are the same as described above.

The term "cancer" as used herein refers to a general term for a disease caused by cells that have aggressive characteristics in which cells divide and proliferate while ignoring normal growth limits, invasive characteristics in which cells infiltrate surrounding tissues, and metastatic characteristics in which cells spread to other parts of the body, and is used with the same meaning as a malignant tumor.

The cancer may be any one selected from the group consisting of, but is not limited to, breast cancer, colon cancer, esophageal cancer, stomach cancer, liver cancer, lung cancer, pancreatic cancer, prostate cancer, gallbladder cancer, bladder cancer, kidney cancer, skin cancer, rectal cancer, osteosarcoma, multiple myeloma, glioma, ovarian cancer, cervical cancer, endometrial cancer, thyroid cancer, laryngeal cancer, testicular cancer, mesothelioma, acute myeloid leukemia, chronic myeloid leukemia, acute lymphoblastic leukemia, chronic lymphoblastic leukemia, brain tumor, neuroblastoma, retinoblastoma, head and neck cancer, salivary gland cancer and lymphoma.

As used herein, the term "treatment" may be used to mean both therapeutic treatment and preventive treatment. In this case, prevention may be used to mean alleviating or reducing a pathological condition or disease of a subject. In one specific embodiment, the term "treatment" includes all applications or any form of medication for treating a disease in a mammal, including a human. In addition, the term includes inhibiting or slowing the progression of a disease; restoring or repairing a damaged or defective function, thereby partially or completely alleviating a disease; or stimulating an ineffective process; or alleviating a serious disease.

In the present specification, the "treatment of cancer" means inhibiting or preventing the growth of cancer cells or tissues, and may also include reducing the growth and metastasis of cancer and reducing resistance to anticancer drugs to enhance the therapeutic effect compared to when no treatment or processing is performed. The cancer metastasis refers to a process in which tumor (cancer) cells spread to distant parts of the body, and "resistance to anticancer drugs" or "anticancer drug resistance" refers to the absence of a therapeutic effect from the beginning of treatment when treating a cancer patient with an anticancer drug, or the initial cancer treatment effect is lost during the continuous treatment process. "Prevention" refers to any act of inhibiting the occurrence of cancer or delaying its onset by administering the pharmaceutical composition.

Pharmacokinetic parameters such as bioavailability and underlying parameters such as clearance rate may also affect efficacy. Therefore, "enhanced efficacy" (e.g., improved efficacy) may be due to improved pharmacokinetic parameters and improved efficacy, and may be measured by comparing parameters such as clearance rate and tumor treatment or improvement in test animals or human subjects.

Here, the term "therapeutically effective amount" or "pharmaceutically effective amount" means an amount of a compound or composition that is effective in preventing or treating a target disease, and is sufficient to treat the disease at a reasonable benefit/risk ratio applicable to medical treatment, and does not cause side effects. The level of the effective amount may be determined based on factors including the patient's health condition, the type and severity of the disease, the activity of the drug, the sensitivity to the drug, the method of administration, the time of administration, the route of administration and the excretion rate, the duration of treatment, drugs used in combination or simultaneously, and other factors well known in the medical field. In one embodiment, the therapeutically effective amount means an amount of a drug that is effective in treating a disease.

In this case, the pharmaceutical composition may further include a pharmaceutically acceptable carrier. The pharmaceutically acceptable carrier may be any non-toxic substance suitable for delivery to a patient. Distilled water, alcohol, fat, wax and inert solids may be included as carriers. A pharmaceutically acceptable adjuvant (buffer, dispersant) may also be included in the pharmaceutical composition.

Specifically, the pharmaceutical composition may be prepared as a parenteral formulation according to the route of administration by a conventional method known in the art, including a pharmaceutically acceptable carrier in addition to the active ingredient. Here, the meaning of "pharmaceutically acceptable" means that it does not inhibit the activity of the active ingredient and does not have toxicity exceeding the adaptability of the application (prescription) target.

In the present invention, the pharmaceutical composition may be administered systemically via parenteral administration. Parenteral administration may be administered in the form of nasal, intranasal, oral, intravenous, intramuscular, intraarterial, intramedullary, intrathecal, intracardiac, transdermal, subcutaneous, intradermal, intraperitoneal, enteral, topical, sublingual or rectal administration, but is not limited thereto.

In the present invention, when the pharmaceutical composition includes a polynucleotide or a vector including (loaded with) the same as an active ingredient, the nucleic acid may be used together with various carriers such as lipid nanoparticles (LNPs), liposomes or vesicles, which are known to effectively deliver polynucleotides into cells, but the present invention is not limited thereto.

The pharmaceutical composition may be prepared in the form of, for example, a powder, a tablet, a capsule, a liquid, an ointment, a cream, a gel, a hydrogel, an aerosol, a spray, a micellar solution, a transdermal patch, a liposomal suspension, a polyplex, an emulsion, a lipid nanoparticle (LNP) (having RNA on its surface or encapsulated therein) or any other suitable form which can be administered to a human or mammal in need of treatment.

In addition, when the pharmaceutical composition is prepared as a parenteral formulation, it may be formulated in the form of injections, transdermal administration, nasal inhalers and suppositories according to a method known in the art together with a suitable carrier. When formulated as an injection, suitable carriers include sterile water, ethanol, polyols such as glycerol or propylene glycol or mixtures thereof, and preferably, Ringer's solution, phosphate buffered saline (PBS) containing triethanolamine, sterile water for injection and isotonic solutions such as 5% dextrose may be used. Regarding the formulation of pharmaceutical compositions, it is known in the art, and specifically, reference can be made to a literature [Remington's Pharmaceutical Sciences (19th ed., 1995)] and the like. The above literature is considered to be a part of the present specification.

The preferred dosage of the pharmaceutical composition may be in a range of 0.01 ug/kg to 10 g/kg or 0.01 mg/kg to 1 g/kg per day, depending on the patient's condition, weight, gender, age, severity of the patient, and route of administration. The administration may be performed once a day or divided into several times. Such dosage should not be construed as limiting the scope of the present invention in any way.

The subject to which the above pharmaceutical composition can be applied (prescribed) may be a mammal, and preferably a human. The pharmaceutical composition of the present invention may additionally include, in addition to the active ingredient, any compound or natural extract known to have anticancer activity.

Still another aspect of the present invention provides a method for preventing or treating cancer, including administering the fusion protein, a polynucleotide encoding the fusion protein or a vector loaded with the polynucleotide to a subject.

Still another aspect of the present invention provides the use of the fusion protein, a polynucleotide encoding the fusion protein or a vector loaded with the polynucleotide in the prevention or treatment of cancer.

The fusion protein, the polynucleotide encoding the fusion protein, the vector loaded with the polynucleotide, administration, prevention and treatment are the same as described above.

In this case, the subject may be a subject suffering from a disease and the like. In addition, the subject may be a mammal, and preferably a human.

The fusion protein or nucleic acid may be administered to a subject in various ways and amounts depending on the patient's condition and the presence or absence of side effects, and the optimal administration method, dosage and administration frequency may be selected within an appropriate range by a person skilled in the art. In addition, the fusion protein or nucleic acid may be administered in combination with other drugs or physiologically active substances known to have therapeutic effects on the disease to be treated, or may be formulated in the form of a combination preparation with other drugs.

### Mutant fragment of E3 ubiquitin ligase

Still another aspect of the present invention provides a βTrCP (beta-transducin repeat-containing protein) fragment or a variant thereof. In addition, still another aspect of the present invention provides a SKP2 (S-phase kinase-associated protein 2) fragment or a variant thereof.

The βTrCP (beta-transducin repeat-containing protein) fragment or variant thereof and SKP2 (S-phase kinase-associated protein 2) fragment or variant thereof are the same as described above.

### MODES OF THE INVENTION

Hereinafter, the present invention will be described in more detail with reference to the following examples. However, the following examples are only intended to illustrate the present invention, and the scope of the present invention is not limited to these examples.

### Example 1. Screening of anti-huSOX2 VHH antibodies

The antigens were prepared by conjugating a 6×His-tag sequence to a C terminus of human SRY-box transcription factor 2 (huSOX2, UniProt: P48431, SEQ ID NO: 1), CynoSOX2 (UniProt: A0A2K5UL35, SEQ ID NO: 2), huSOX3 (UniProt: P41225, SEQ ID NO: 3) and the 135-317 fragment of huSOX2 (huSOX2₁₃₅₋₃₁₇, SEQ ID NO: 4) and requesting production by Biointron.

Specifically, the protein was expressed in *E. coli* cultured in 4 L, purified by using His-tag, and then harvested. Other antigens were purchased from the relevant vendors and used.

The discovery of nanobodies that bind to huSOX2 was commissioned to Elpis Biotech, and 4 rounds of panning were conducted by using huSOX2 (Cusabio, Cat no. CSB-EP022426HU(M)) antigen. As a result, huSOX2-specific VHH antibody S14 (SEQ ID NO: 5) was obtained.

Panning was performed by using the VHH phage library. huSOX2₁₃₅₋₃₁₇ protein produced by Biointron was added to an immunotube at a concentration of 20 ug/mL, and the protein was adsorbed to the surface of the tube for 1 hour. Then, 3% non-fat milk solution was added to the tube to protect the surface such that huSOX2₁₃₅₋₃₁₇ was not adsorbed. After emptying the tube, 2 × 10¹¹ CFU of antibody phage library dispersed in 3% non-fat milk solution was added thereto and reacted with the antigen. Non-specifically bound phage were washed three times with 1 × PBST (phosphate-buffered saline + Tween20) buffer solution. The remaining antigen-specific phage antibodies were eluted with a 100 mM triethylamine (Sigma, Cat no. 471283) solution. The eluted phage was neutralized with 1.0 M Tris-HCl (Bioworld, TR2016-050-75) buffer and then transformed into TG1 (Lucigen, Cat no. 60502-2*) E. coli* to measure the titer. The phage was amplified in the same host before the next round of panning.

The amplified *E. coli* was suspended in 5 mL of SB medium (20 g Yeast extract (Duchfa, Cat no. T1333), 30 g Trypton (Duchfa, Cat no. T1332), 10 g MOPS (Duchefa, Cat no. M1502) and 100 ug/mL ampicillin (Biosesang, Cat no. AC1043-025-00)/1 L distilled water). 50 µL of the suspension was inoculated into 20 mL of SB-ampicillin solution and cultured at 37°C. When the absorbance (OD₆₀₀) of the culture became 0.5, 10¹⁰ PFU of M13KO7 helper phage (Invitrogen, Cat no. 18311-019) was added and cultured at 37°C with gentle stirring. After 1 hour, 70 ug/mL of kanamycin (Biosesang, Cat no. KC1001-025-02) was added and cultured overnight at 30°C with rapid stirring (220 rpm).

The next day, the culture medium was centrifuged, and the supernatant including the phage particles was transferred to a new 50 mL tube, and 5 mL of PEG/NaCl solution was added and reacted on ice for 30 minutes. The precipitated phage was centrifuged and dissolved in 1 × PBS buffer. The phage solution was used as a library and the panning process was repeated to enrich antigen-specific clones.

Individual colonies that underwent panning were subjected to 96-well ELISA screening for 10 ug/mL off-target antigen, 20 ug/mL huSOX2 and huSOX2₁₃₅₋₃₁₇ proteins. The sequences of clones that showed negative signals for the off-target antigen proteins and positive signals for huSOX2 proteins (two types) were identified, and 9 clones (SEQ ID NOs: 6 to 14) with different sequences were derived.

Anti-huSOX2 VHH antibodies were screened from a single domain antibody (sdAb) naive phage display library by using huSOX2 (SEQ ID NO: 1), cynoSOX2 (SEQ ID NO: 2) and huSOX3 (SEQ ID NO: 3) produced by Biointron at the request of Genscript Probio as antigens, respectively. After three rounds of panning, 3F11 (SEQ ID NO: 15), 3A2 (SEQ ID NO: 16), and 3H9 (SEQ ID NO: 17) (a total of three) were selected as anti-huSOX2 VHH antibodies that bound to huSOX2 and cynoSOX2, but not to huSOX3.

As a result of analyzing the sequences of anti-huSOX2 VHH antibodies obtained through the antibody selection using the above three methods, 13 clones having the amino acid sequences described in FIG. 1 and Table 2 were finally derived.

**[Table 2]**

| **Clone Name** | FR1 | HCDR1 | FR2 | HCDR2 | FR3 | HCDR3 | FR4 |
|---|---|---|---|---|---|---|---|
| S14 (SEQ ID NO: 5) | QVKLEESGG GSVQTGGSL RLTCAASG (SEQ ID NO: 55) | GSIFSMNRY (SEQ ID NO: 56) | MGWFRQAP GKERELVAV (SEQ ID NO: 57) | IDSKRNKT (SEQ ID NO: 58) | HYADSVKGRFT ISRDNAAKNTVD LQMNSLKPEDT AIYYC (SEQ ID NO: 59) | IAWTGKPPRGY (SEQ ID NO: 60) | WGQGTQVTVSS (SEQ ID NO: 61) |
| 1A5 (SEQ ID NO: 6) | EVQLVESGG GLVQPGGSL RLSCAASG (SEQ ID NO: 62) | GSTYTTYS (SEQ ID NO: 63) | MGWFRQAP GKEREFVGAA (SEQ ID NO: 64) | IHWSGGRT (SEQ ID NO: 65) | YYADSVKGRFT ISADNSKNTAY LQMNSLKPEDT AVYYC (SEQ ID NO: 66) | ASRVDDRVG (SEQ ID NO: 67) | WGQGTLVTVSS (SEQ ID NO: 68) |
| 1A9 (SEQ ID NO: 7) | EVQLVESGG GLVQPGGSL RLSCAASG (SEQ ID NO: 62) | GRTFSTYS (SEQ ID NO: 69) | MGWFRQAP GKEREFVAA (SEQ ID NO: 70) | IRWTAGTT (SEQ ID NO: 71) | YYADSVKGRFT ISADNSKNTAY LQMNSLKPEDT AVYYC (SEQ ID NO: 66) | ASRVDDRVG (SEQ ID NO: 67) | WGQGTLVTVSS (SEQ ID NO: 68) |
| 1A11 (SEQ ID NO: 8) | EVQLVESGG GLVQPGGSL RLSCAASG (SEQ ID NO: 62) | GSTYTTYS (SEQ ID NO: 63) | MGWFRQAP GKEREFVAA (SEQ ID NO: 70) | IHWSGGGT (SEQ ID NO: 72) | YYADSVKGRFT ISADNSKNTAY LQMNSLKPEDT AVYYC (SEQ ID NO: 66) | ASRVDDRVG (SEQ ID NO: 67) | WGQGTLVTVSS (SEQ ID NO: 68) |
| 1B9 (SEQ ID NO: 9) | EVQLVESGG GLVQPGGSL RLSCAASG (SEQ ID NO: 62) | GLTFSTYS (SEQ ID NO: 73) | MGWFRQAP GKEREFVAA (SEQ ID NO: 70) | ITWNSGRRT (SEQ ID NO: 74) | YYADSVKGRFT ISADNSKNTAY LQMNSLKPEDT AVYYC (SEQ ID NO: 66) | ASRVDDRVG (SEQ ID NO: 67) | WGQGTLVTVSS (SEQ ID NO: 68) |
| 1C2 (SEQ ID NO: 10) | EVQLVESGG GLVQPGGSL RLSCAASG (SEQ ID NO: 62) | GSTYTTYS (SEQ ID NO: 63) | MGWFRQAP GKEREFVAA (SEQ ID NO: 70) | IRWSGGTT (SEQ ID NO: 75) | YYADSVKGRFT ISADNSKNTAY LQMNSLKPEDT AVYYC (SEQ ID NO: IN) | ASRVDDRVG (SEQ ID NO: 67) | WGQGTLVTVSS (SEQ ID NO: 68) |
| 1C11 (SEQ ID NO: 11) | EVQLVESGG GLVQPGGSL RLSCAASG (SEQ ID NO: 62) | GSTYTTYS (SEQ ID NO: 63) | MGWFRQAP GKEREFVGAA (SEQ ID NO: 64) | IRWSGGTT (SEQ ID NO: 75) | YYADSVKGRFT ISADNSKNTAY LQMNSLKPEDT AVYYC (SEQ ID NO: 66) | ASRVDDRVG (SEQ ID NO: 67) | WGQGTLVTVSS (SEQ ID NO: 68) |
| 1C12 (SEQ ID NO: 12) | EVQLVESGG GLVQPGGSL RLSCAASG (SEQ ID NO: 62) | GSTYTTYS (SEQ ID NO: 63) | MGWFRQAP GKEREFVAA (SEQ ID NO: 70) | IRWSGGST (SEQ ID NO: 76) | HYADSVKGRFT ISADNSKNTAY LQMNSLKPEDT AVYYC (SEQ ID NO: 77) | ASRVDDRVG (SEQ ID NO: 67) | WGQGTLVTVSS (SEQ ID NO: 68) |
| 2E8 (SEQ ID NO: 14) | EVQLVESGG GLVQPGGSL RLSCAASG (SEQ ID NO: 62) | GFTLDYYG (SEQ ID NO: 78) | MGWFRQAP GKEREFVAA (SEQ ID NO: 70) | ISRSGGST (SEQ ID NO: 79) | YYADSVKGRFT ISADNSKNTAY LQMNSLKPEDT AVYYC (SEQ ID NO: 80) | AREVVRGGI NY (SEQ ID NO: 81) | WGQGTLVT VSS (SEQ ID NO: 68) |
| 1C4 (SEQ ID NO: 13) | EVQLVESGG GLVQPGGSL RLSCAASG (SEQ ID NO: 62) | GGGFRGNAV (SEQ ID NO: 82) | MGWFRQAP GKERELVAA (SEQ ID NO: 83) | IRGSGEDT (SEQ ID NO: 84) | YYADSVKGRFT ISADNSKNTAY LQMNSLKPEDT AVYYC (SEQ ID NO: 66) | AGGWRRGYF QD (SEQ ID NO: 85) | WGQGTLVT VSS (SEQ ID NO: 68) |
| 3F11 (SEQ ID NO: 15) | DVQLVESGG GLVQPGGSL RLSCATS (SEQ ID NO: 86) | GFTFSSST (SEQ ID NO: 87) | MSWVRQVP GAGPEWVAV (SEQ ID NO: 88) | DNPGGKK (SEQ ID NO: 89) | NYIDSVAGRFT IARDNDANSLS LVMNGLRPEDT ARYFC (SEQ ID NO: 90) | ARVLSFKIY EGDKWALEW DY (SEQ ID NO: 91) | RGPGTQVT VSS (SEQ ID NO: 92) |
| 3A2 (SEQ ID NO: 16) | EVQLVESGG EQVQPGSSL KLSCSVS (SEQ ID NO: 93) | GLMISGHA (SEQ ID NO: 94) | MSWVRQVP GAGPEWVAV (SEQ ID NO: 88) | DNPGGKK (SEQ ID NO: 89) | NYIDSVAGRFT IARDNDANSLS LVMNGLRPEDT ARYFC (SEQ ID NO: 90) | ARVLSFKIY EGDKWALEW DY (SEQ ID NO: 91) | RGPGTQVT VSS (SEQ ID NO: 92) |
| 3H9 (SEQ ID NO: 17) | QVQLVESGG EQVQPGSSL KLSCSVS (SEQ ID NO: 95) | GLMISGHA (SEQ ID NO: 94) | MSWVRQVP GAGPEWVAV (SEQ ID NO: 88) | DNPGGKK (SEQ ID NO: 89) | NY IDSVAGRFT IARDNDANSLS LVMNGLRPEDT ARYFC (SEQ ID NO: 90) | ARVLSFKIY GGDKWALEW DY (SEQ ID NO: 96) | RGPGTQVT VSS (SEQ ID NO: 92) |

### Example 2. Confirmation of binding affinity of selected anti-huSOX2 VHH antibody clones

9 of the 13 selected clones were selected and inserted into pYD5, which is a yeast surface expression vector constructed with reference to a research article (Wang Z et al., "A new yeast display vector permitting free scFv amino termini can augment ligand binding affinities". Protein Eng Des Sel. (2005) 18:337-343, doi:10.1093/protein/gzi036).

The clones were cultured at 30°C for approximately 16 hours by using SD-CAA medium (20 g glucose (SIGMA, Cat no. G7528), 14.7 g sodium citrate (SIGMA, Cat no. C8532), 4.3 g citric acid monohydrate (SIGMA, Cat no. C0706), 6.7 g yeast nitrogen base (BD Difco, Cat no. 291940), 5 g bacto casamino acid (BD Difco, Cat no. 223050), 100 ug/mL kanamycin (Biosesang, Cat no. KC1001-025-02)/1 L distilled water). The culture was diluted to OD₆₀₀ = 1 in SG-CAA medium and additionally cultured at 30°C for 18 hours to induce the expression of anti-huSOX2 VHH antibodies on the yeast surface.

Primary staining was performed by mixing 0.5× 10⁷ cultured yeast, 2 µM biotinylated huSOX2 (Biointron) or 2 µM biotinylated off-target antigen (human recombinant SKP1 protein, Sinobiological, Cat no. 14161-H40E-B), and 1:500 anti-V5 mouse antibody (Invitrogen, Cat no. R960-25) (diluted 1:500 in wash buffer (PBS containing 0.1% BSA)) and reacting for 30 minutes at room temperature.

Then, Streptavidin, R-Phycoerythrin Conjugate (SAPE) (Invitrogen, Cat no. SA10044) and anti-mouse IgG (H + L)-FITC antibody (Invitrogen, Cat no. 11-4011-85), which were diluted in wash buffer at a ratio of 1:100, were added, and the mixture was incubated in the dark at 4°C for 20 minutes (secondary staining). The reaction product was analyzed by using a flow cytometer (SONY, SH800S) to confirm the binding affinity of the anti-huSOX2 VHH antibody to the huSOX2 protein.

As a result, as shown in FIGS. 2a and 2b, it was confirmed that the anti-huSOX2 antibody produced as described above specifically binds only to SOX2.

### Example 3. Production of anti-huSOX2 VHH antibodies and antigen binding assay

The affinity of the antigen for anti-huSOX2 VHH antibodies (3 types; 3A2, 3F11, 3H9) produced by the method of Example 1 above was evaluated.

First of all, a single domain antibody (sdAb) in the form of a human Fc (SEQ ID NO: 151) conjugated to codon-optimized human Fc was expressed in Expi-CHO-S cells, and then, sdAb-Fc was purified by using a Protein A affinity column (Table 3). The binding affinity of each antibody to the huSOX2 protein was confirmed by using a Surface Plasmon Resonance (SPR) biosensor (Biacore 8K).

**[Table 3]**

| Name | | Sequence | SEQ ID NO: |
|---|---|---|---|
| 3F112-Fc | Fc | | 151 |
| | linker | GGGGSGGGGSGGGGS | 238 |
| | 3F11 | DVQLVESGGGLVQPGGSLRLSCATSGFTFSSSTMSWVRQVPGAGPEW VAVDNPGGKKNYIDSVAGRFTIARDNDANSLSLVMNGLRPEDTARY FCARVLSFKIYEGDKWALEWDYRGPGTQVTVSS | 15 |
| 3A2-Fc | Fc | | 151 |
| | linker | GGGGSGGGGSGGGGS | 238 |
| | 3A2 | EVQLVESGGEQVQPGSSLKLSCSVSGLMISGHAMSWVRQVPGAGPE WVAVDNPGGKKNYIDSVAGRFTIARDNDANSLSLVMNGLRPEDTAR YFCARVLSFKIYEGDKWALEWDYRGPGTQVTVSS | 16 |
| 3H9-Fc | Fc | | 151 |
| | linker | GGGGSGGGGSGGGGS | 238 |
| | 3H9 | QVQLVESGGEQVQPGSSLKLSCSVSGLMISGHAMSWVRQVPGAGPE WVAVDNPGGKKNYIDSVAGRFTIARDNDANSLSLVMNGLRPEDTAR YFCARVLSFKIYGGDKWALEWDYRGPGTQVTVSS | 17 |

Specifically, the antibody was captured to about 100 RU on the sensor chip Protein A via the Fc capture method. The huSOX2 protein was diluted as an analyte and injected onto the surface of flows 1 and 2, followed by injection of the running buffer as a dissociation step. The dissociation rate constant (kd) and association rate constant (ka) were analyzed by using Biacore 8K evaluation software. In addition, the equilibrium dissociation constant (KD) was calculated from the ratio of kd to ka. The results of the analysis are shown in Table 4.

**[Table 4]**

| Type of antibody | 3F11 | 3A2 | 3H9 |
|---|---|---|---|
| Antigen | huSOX2 | huSOX2 | huSOX2 |
| ka(1/Ms) | 1.75E+05 | 1.48E+05 | 2.13E+05 |
| kd(1/s) | 5.41E-04 | 3.52E-04 | 2.26E-04 |
| KD(M) | 2.12E-09 | 2.39E-09 | 1.06E-09 |

### Example 4. Confirmation of specific binding affinity of anti-huSOX2 VHH antibodies and target proteins

Among the single domain antibody clones selected in Example 1 above, 5 types were used to measure the specific binding affinity for huSOX2 protein using NanoBRET^{™}Nano-Glo^{®} substrate (Promega, Cat no. N1661).

Specifically, the expression vector for the huSOX2 protein was constructed by sequentially loading a polynucleotide encoding a Halo tag (SEQ ID NO: 154) and a polynucleotide encoding huSOX2 (SEQ ID NO: 97) at the N-terminus into the pHTN vector (Promega, Cat no. G7721). In addition, the expression vectors of each of the 5 single domain antibody clones were constructed by sequentially loading a NanoLuc tag (SEQ ID NO: 156) and the polynucleotides encoding the 5 single domain antibody clones (SEQ ID NO: 101, SEQ ID NO: 110, SEQ ID NO: 111, SEQ ID NO: 112, or SEQ ID NO: 113) at the N-terminus into the pNLF1 vector (Promega, Cat no. N1351), respectively, to construct expression vectors (pNLF1-N-S14, pNLF1-N-2E8, pNLF1-N-3F11, pNLF1-N-3A2, pNLF1-N-3H9).

The expression vectors produced as above were combined at each ratio and transfected into a human kidney cell line (HEK293T cells) using Lipofectamine 3000 (Lipofectamine 3000, Invitrogen, Cat no. L3000001). 24 hours after transfection, HEK293T cells were treated with HaloTag 618, which is a ligand that binds to the Halo tag, and cultured for additional 6 hours. Afterwards, the cells were treated with Nano-Glo substrate to induce luminescence, and the luminescence value was measured by using a microplate reader (BioTek, Synergy HTX). The results were graphed by using Graphpad prism software.

As a result, as shown in FIG. 3, it was confirmed that all 5 antibody clones used specifically bind to the huSOX2 protein.

### Example 5. Production of BIOPROTACS including anti-huSOX2 VHH antibodies

### Example 5.1. Production of fusion proteins including S14 and E3 ubiquitin ligase fragments

In order to determine the SOX2 protein-specific degradation capability of a fusion protein including the selected single domain antibody S14 and E3 ubiquitin ligase, a fusion protein including the E3 ubiquitin ligase fragment and S14 was produced. In this case, the amino acid sequences of the ubiquitin ligase fragments (11 types) used are listed in Table 5.

The fusion protein was produced in a form including E3 ubiquitin ligase fragment (SEQ ID NO: 18, SEQ ID NO: 19, SEQ ID NO: 24, SEQ ID NO: 25 or SEQ ID NO: 27)-GS (SEQ ID NO: 157)-S14 (SEQ ID NO: 5) or S14 (SEQ ID NO: 5)-GS (SEQ ID NO: 157)-E3 ubiquitin ligase fragment (SEQ ID NO: 20 to SEQ ID NO: 23, SEQ ID NO: 26 or SEQ ID NO: 28), and a Flag-tag (SEQ ID NO: 162) was included at the N-terminus of the E3 ubiquitin ligase fragment or S14 located at the N-terminus. The polynucleotides (SEQ ID NO: 129 to SEQ ID NO: 139) encoding the fusion protein were each loaded onto a pCMV6 (Origene, Cat no. PS100001) vector for mammalian cell expression. The sequence of the fusion protein is described in Table 6. Hereinafter, the fusion protein including the S14 and E3 ubiquitin ligase fragments is described interchangeably as "S14-E3 ubiquitin ligase."

An expression vector was constructed by loading a polynucleotide (SEQ ID NO: 128) encoding huSOX2.Myc (SEQ ID NO: 32) including a Myc tag (SEQ ID NO: 158) at the C-terminus into the vector.

**[Table 5]**

| Name | UniProt ID. | Position | Sequence | SEQ ID NO: |
|---|---|---|---|---|
| βTrCP₁₉₀₋₂₂₈ | Q9Y297 | 190-228 | DPAEAVLQEKALKFMNSSEREDCNNGEPPRKIIPEK NSLRQTYNSCARLCLNQETVCLASTAMKTENCVA KTKLANGTSSMIVPKQRKLSASYEKEKELCVKYFE QWSESDQVEFVEHLISQMCHYQHGHINSYLKPMLQ RDFITALPARGLDHIAENILSYLDAKSLCAAELVCK EWYRVTSDGMLWKKLIERMVRTDSLWRGLAERR GWGQYLFKNKPPDGNAPPNSFYRALYPKIIQDIETI ESNWRCGRHSLQRIHCR | 18 |
| SKP2₂₋₁₇₆ | Q13309 | 2-176 | HRKHLQEIPDLSSNVATSFTWGWDSSKTSELLSGM GVSALEKEEPDSENIPQELLSNLGHPESPPRKRLKSK GSDKDFVIVRRPKLNRENFPGVSWDSLPDELLLGIF SCLCLPELLKVSGVCKRWYRLASDESLWQTLDLTG KNLHPDVTGRLLSQGVIAFRCPRSFMDQPLAE | 19 |
| VHL₁₅₂₋₂₁₃ | P40337 | 152-213 | TLPVYTLKERCLQVVRSLVKPENYRRLDIVRSLYE DLEDHPNVQKDLERLTQERIAHQRMGD | 20 |
| SPOP₁₆₇₋₃₇₄ | 043791 | 167-374 | | 21 |
| SOCS2₁₄₃₋₁₉₈ | 014508 | 143-198 | | 22 |
| CHIP₁₂₈₋₃₀₃ | Q9UNE7 | 128-303 | RLNFGDDIPSALRIAKKKRWNSIEERRIHQESELHSY LSRLIAAERERELEECQRNHEGDEDDSHVRAQQAC IEAKHDKYMADMDELFSQVDEKRKKRDIPDYLCG KISFELMREPCITPSGITYDRKDIEEHLQRVGHFDPV TRSPLTQEQLIPNLAMKEVIDAFISENGWVEDY | 23 |
| DDB2₂₋₁₁₄ | Q92466 | 2-114 | APKKRPETQKTSEIVLRPRNKRSRSPLELEPEAKKL CAKGSGPSRRCDSDCLWVGLAGPQILPPCRSIVRTL HQHKLGRASWPSVQQGLQQSFLHTLDSYRILQKAA PFDRRA | 24 |
| CRBN₂₋₃₂₀ | Q96SW2 | 2-320 | AGEGDQQDAAHNMGNHLPLLPAESEEEDEMEVED QDSKEAKKPNIINFDTSLPTSHTYLGADMEEFHGRT LHDDDSCQVIPVLPQVMMILIPGQTLPLQLFHPQEV SMVRNLIQKDRTFAVLAYSNVQEREAQFGTTAEIY AYREEQDFGIEIVKVKAIGRQRFKVLELRTQSDGIQ QAKVQILPECVLPSTMSAVQLESLNKCQIFPSKPVS REDQCSYKWWQKYQKRKFHCANLTSWPRWLYSL YDAETLMDRIKKQLREWDENLKDDSLPSNPIDFSY RVAACLPIDDVLRIQLLKIGSAIQRLRCELDIMNKCT S | 25 |
| ASB1₂₆₆₋₃₃₅ | Q9Y576 | 266-335 | VKWESLGPESRGRRKVDPEALQVFKEARSVPRTLL CLCRVAVRRALGKHRLHLIPSLPLPDPIKKFLLHE | 26 |
| TRIM21₂₋₈₅ | P19474 | 2-85 | | 27 |
| RNF4₇₁₋₁₉₀ | P78317 | 71-190 | DERRRPRRNARRLPQDHADSCVVSSDDEELSRDRD VYVTTHTPRNARDEGATGLRPSGTVSCPICMDGYS EIVQNGRLIVSTECGHVFCSQCLRDSLKNANTCPTC RKKINHKRYHPIYI | 28 |

**[Table 6]**

| **Name** | **Fusion protein** | **Sequence** | **SEQ ID NO:** |
|---|---|---|---|
| S14.001 (SEQ ID NO: 33) | Flag-tag | DYKDDDDK | 162 |
| | βTrCP₁₉₀₋₂₂₈ | DPAEAVLQEKALKFMNSSEREDCNNGEPPPKIIPEKNSLRQTYNSCARLCLNQ ETVCLASTAMKTENCVAKTKLANGTSSMIVPKQRKLSASYEKEKELCVKYFEQ WSESDQVEFVEHLISQWCHYQHGHINSYLKPMLQRDFITALPARGLDHIAENI LSYLDAKSLCAAELVCKEWYRVTSDGMLWKKLIERWVRTDSLWRGLAERRGWG QYLFKNKPPDGNAPPNSFYRALYPKIIQDIETIESNWRQGRHSLQRIHCR | 18 |
| | linker | GS | 157 |
| | S14 | QVKLEESGGGSVQTGGSLRLTCAASGSIFSMNRMGWFRQAPGKERELVAVIDS KRNKTHYADSVKGRFTISRDNAKNTVDLQMNSLKPEDTAIYYCTAWTGRPPRG YWGQGTQVTVSS | 5 |
| S14.003 (SEQ ID NO: 34) | Flag-tag | DYKDDDDK | 162 |
| | SKP₂₂₋₁₇₆ | HRKHLQEIPDLSSNVATSFTWGWDSSKTSELLSGMGVSALEKEEPDSENIPQE LLSNLGHPESPPPKKRLKSKGSDKDFVIVRRPKLNRENFPGVSWDSLPDELLLG IFSCLCLPELLKVSGVCKRWYRLASDESLWQTLDLTGKNLHPDVTGRLLSQGV IAFRCPRSFMDQPLAE | 19 |
| | linker | GS | 157 |
| | S14 | QVKLEESGGGSVQTGGSLRLTCAASGSIFSMNRMGWFRQAPGKERELVAVIDS KRNKTHYADSVKGRFTISRDNAKNTVDLQMNSLKPEDTAIYYCTAWTGRPPRG YWGQGTQVTVSS | 5 |
| S14.004 (SEQ ID NO: 35) | Flag-tag | DYKDDDDK | 162 |
| | S14 | QVKLEESGGGSVQTGGSLRLTCAASGSIFSMNRMGWFRQAPGKERELVAVIDS KRNKTHYADSVKGRFTISRDNAKNTVDLQMNSLKPEDTAIYYCTAWTGRPPRG YWGQGTQVTVSS | 5 |
| | linker | GS | 157 |
| | VHL₁₅₂₋₂₁₃ | TLPVYTLKERCLQVVRSLVKPENYRRLDIVRSLYEDLEDIIPNVQKDLERLTQE RIAIIQRMGD | 20 |
| S14.005 (SEQ ID NO: 36) | Flag-tag | DYKDDDDK | 162 |
| | S14 | QVKLEESGGGSVQTGGSLRLTCAASGSIFSMNRMGWFRQAPGKERELVAVIDS KRNKTHYADSVKGRFTISRDNAKNTVDLQMNSLKPEDTAIYYCTAWTGRPPRG YWGQGTQVTVSS | 5 |
| | linker | GS | 157 |
| | SPOP₁₆₇₋₃₇₄ | SVNISGQNTMNMVKVPECRLADELGGLWENSRFTDCCLCVAGQEFQAHKAILA ARSPVFSAMFEIHEMEESKKNRVEINDVEPEVFKEMMCFIYTGKAPNLDKMADD LLAAADKYALERLKVMCEDALCSNLSVENAAEILILADLHSADQLKTQAVDFI NYHASDVLETSGWKSMVVSIPHLVAEAYRSLASAQCPFLGPPRRKLKQS | 21 |
| S14.006 (SEQ ID NO: 37) | Flag-tag | DYKDDDDK | 162 |
| | S14 | QVKLEESGGGSVQTGGSLRLTCAASGSIFSMNRMGWFRQAPGKERELVAVIDS KRNKTHYADSVKGRFTISRDNAKNTVDLQMNSLKPEDTAIYYCTAWTGRPPRG YWGQGTQVTVSS | 5 |
| | linker | GS | 157 |
| | SOCS2₁₄₃₋₁₉₈ | PRNGTVHLYLTKPLYTSAPSLQHLLCRLTNKCTGAIWGLPLPTRLKDYLEEYK FQV | 22 |
| S14.007 (SEQ ID NO: 38) | Flag-tag | DYKDDDDK | 162 |
| | S14 | QVKLEESGGGSVQTGGSLRLTCAASGSIFSMNRMGWFRQAPGKERELVAVIDS KRNKTHYADSVKGRFTISRDNAKNTVDLQMNSLKPEDTAIYYCTAWTGRPPRG YWGQGTQVTVSS | 5 |
| | linker | GS | 157 |
| | CHIP₁₂₆₋₃₀₃ | RLNFGDDIPSALRIAKKKRWNSIEERRIHQESELIHSYLSRLIAAERERELEEC QRNHEGDEDDSHVRAQQACIEAKHDKYMADMDELFSQYDEKRKKRDIPDYLQG KISFELMREPCITPSGITYDRKDIEEHLQRVGHFDPVTRSPLTQEQLIPNLAM KEVIDAFISENGWVEDY | 23 |
| S14.008 (SEQ ID NO: 39) | Flag-tag | DYKDDDDK | 162 |
| | DDB2₂₋₁₁₄ | APKKRPETQKTSEIVLRPRNKRSRSPLELEPEAKKLCAKGSGPSRRCDSDCLW VGLAGPQILPPCRSIVRTLHQHKLGRASWPSVQQGLQQSFLHTLDSYRILQKA APFDRRA | 24 |
| | linker | GS | 157 |
| | S14 | QVKLEESGGGSVQTGGSLRLTCAASGSIFSMNRMGWFRQAPGKERELVAVIDS KRNKTHYADSVKGRFTISRDNAKNTVDLQMNSLKPEDTAIYYCTAWTGRPPRG YWGQGTQVTVSS | 5 |
| S14.009 (SEQ ID NO: 40) | Flag-tag | DYKDDDDK | 162 |
| | CRBN₂₋₂₃₀ | AGEGDQQDAAHNMGNHLPLLPAESEEEDEMEVEDQDSKEAKKPNIINFDTSLP TSHTYLGADWEEPHGRTLHDDISCQVIPVLPQVMYLLIPGQTLPLQLFHPQEV SNVRNLIQKDRTFAVLAYSNVQEREAQFGTTAEIYAYREEDDFGIEIVKVKAI GRQRFKVLELRTQSDGIQQAKVQLLPECVLPSTMSAVQLESLNKCQIFPSKPV SREDQCSYKWWQKYQKRKFHCANLTSWPRWLYSLYDAETLMDRIKKQLREWDE NLKDDSLPSNPIDFSYRVAACLPIDDVLRIQLLKIGSAIQRLRCELDIMNKCTS | 25 |
| | linker | GS | 157 |
| | S14 | QVKLEESGGGSVQTGGSLRLTCAASGSIFSMNRMGWFRQAPGKERELVAVIDS KRNKTHYADSVKGRFTISRDNAKNTVDLQMNSLKPEDTAIYYCTAWTGRPPRG YWGQGTQVTVSS | 5 |
| S14.010 (SEQ ID NO: 41) | Flag-tag | DYKDDDDK | 162 |
| | S14 | QVKLEESGGGSVQTGGSLRLTCAASGSIFSMNRMGWFRQAPGKERELVAVIDS KRNKTHYADSVKGRFTISRDNAKNTVDLQMNSLKPEDTAIYYCTAWTGRPPRG YWGQGTQVTVSS YWGQGTQVTVSS | 5 |
| | linker | GS | 157 |
| | ASB1₂₆₆₋₃₃₅ | VKWESLGPESRGRRKVDPEALQVFKEARSVPRTLLCLCRVAVRRALGKHRLHL IPSLPLPDPPIKKFLLHE | 26 |
| S14.011 (SEQ ID NO: 42) | Flag-tag | DYKDDDDK | 162 |
| | TRIM21₂₋₈₅ | ASAARLTAMWEEVTCPICLDPFVEPVSIECGHSFCQECISQVGKGGGSVCPVC RQRFLLKNLRPNRQLANMVNNLKEISQEARE | 27 |
| | linker | GS | 157 |
| | S14 | QVKLEESGGGSVQTGGSLRLTCAASGSIFSMNRMGWFRQAPGKERELVAVIDS KRNKTHYADSVKGRFTISRDNAKNTVDLQMNSLKPEDTAIYYCTAWTGRPPRG YWGQGTQVTVSS | 5 |
| S14.012 (SEQ ID NO: 43) | Flag-tag | DYKDDDDK | 162 |
| | S14 | QVKLEESGGGSVQTGGSLRLTCAASGSIFSMNRMGWFRQAPGKERELVAVIDS KRNKTHYADSVKGRFTISRDNAKNTVDLQMNSLKPEDTAIYYCTAWTGRPPRG YWGQGTQVTVSS | 5 |
| | linker | GS | 157 |
| | RNF4₇₁₋₁₉₀ | DERRRPRRNARRLPQDHADSCVVSSDDEELSRDRDVYVTTHTPRNARDEGATG LRPSGTVSCPICMDGYSEIVQNGRLIVSTECGHWFCSQCLRDSLKNANTCPTC RKKINHKRYHPIYI | 28 |

### Example 5.2. Production of fusion proteins including 3A2 and E3 ubiquitin ligase fragments

In order to confirm the SOX2 protein-specific degradation capability of the fusion protein including the single domain antibody 3A2 and the E3 ubiquitin ligase fragment selected in Example 1 above, a fusion protein including the E3 ubiquitin ligase fragment and 3A2 was produced. In this case, the amino acid sequences of the ubiquitin ligase fragments (10 types) used are described in Table 7.

The fusion protein includes 3A2 (SEQ ID NO: 16)-GS (SEQ ID NO: 157)-E3 ubiquitin ligase fragment (SEQ ID NO: 20 to SEQ ID NO: 23, SEQ ID NO: 26, or SEQ ID NO: 28 to SEQ ID NO: 31), or E3 ubiquitin ligase fragment (SEQ ID NO: 27)-GS (SEQ ID NO: 157)-3A2 (SEQ ID NO: 16), and was produced in a form in which a Flag-tag is included at the N-terminus of the 3A2 or E3 ubiquitin ligase fragment located at the N-terminus.

The polynucleotides (SEQ ID NOs: 140 to 149) encoding the fusion proteins were each loaded onto the pCMV6 (Origene, Cat no. PS100001) vector for mammalian cell expression. The sequences of the fusion proteins are described in Table 8. Hereinafter, the fusion proteins including the 3A2 and E3 ubiquitin ligase fragments are described interchangeably with "3A2-E3 ubiquitin ligase".

An expression vector was constructed by loading a polynucleotide (SEQ ID NO: 150) encoding huSOX2.V5 (SEQ ID NO: 54) including a V5-tag (SEQ ID NO: 160) at the C-terminus into the vector.

**[Table 7]**

| Name | UniProt ID. | Position | Sequence | SEQ ID NO: |
|---|---|---|---|---|
| VHL₁₅₂₋₂₁₃ | P40337 | 152-213 | TLPVYTLKERCLQVVRSLVKPENYRRLDIVRSLYED LEDIIPNVQKDLERLTQERIAIIQRMGD | 20 |
| SPOP₁₆₇₋₃₇₄ | 043791 | 167-374 | | 21 |
| SOCS2₁₄₃₋₁₉₈ | 014508 | 143-198 | | 22 |
| CHIP₁₂₈₋₃₀₃ | Q9UNE7 | 128-303 | RLNFGDDIPSALRIAKKKRWNSIEERRIHQESELHSY LSRLIAAERERELEECQRNHEGDEDDSHVRAQQACI EAKHDKYMADMDELFSQVDEKRKKRDIPDYLCGKI SFELMREPCITPSGITYDRKDIEEHLQRVGHFDPVTRS PLTQEQLIPNLAMKEVIDAFISENGWVEDY | 23 |
| ASB1₂₆₆₋₃₃₅ | Q9Y576 | 266-335 | VKWESLGPESRGRRKVDPEALQVFKEARSVPRTLLC LCRVAVRRALGKHRLHLIPSLPLPDPIKKFLLHE | 26 |
| TRIM21₂₋₈₅ | P19474 | 2-85 | | 27 |
| RNF4₇₁₋₁₉₀ | P78317 | 71-190 | DERRRPRRNARRLPQDHADSCVVSSDDEELSRDRD VYVTTHTPRNARDEGATGLRPSGTVSCPICMDGYSE IVQNGRLIVSTECGHVFCSQCLRDSLKNANTCPTCR KKINHKRYHPIYI | 28 |
| UBE2D1₂₋₁₄₇ | P51668 | 2-147 | ALKRIQKELSDLQRDPPAHCSAGPVGDDLFHWQATI MGPPDSAYQGGVFFLTVHFPTDYPFKPPKIAFTTKIY HPNINSNGSICLDILRSQWSPALTVSKVLLSICSLLCD PNPDDPLVPDIAQIYKSDKEKYNRHAREWTQKYAM | 29 |
| UBE2D4₂₋₁₄₇ | Q9Y2X8 | 2-147 | ALKRIQKELTDLQRDPPAQCSAGPVGDDLFHWQATI MGPNDSPYQGGVFFLTIHFPTDYPFKPPKVAFTTKIY HPNINSNGSICLDILRSQWSPALTVSKVLLSICSLLCD PNPDDPLVPEIAHTYKADREKYNRLAREWTQKYAM | 30 |
| ZNFR1₁₃₈₋₂₂₇ | Q8ND25 | 138-227 | SSHSGFKCPICSKSVASDEMEMHFIMCLSKPRLSYND DVLTKDAGECVICLEELLQGDTIARLPCLCIYHKSCI DSWFEVNRSCPEHPAD | 31 |

**[Table 8]**

| **Name** | **Constitution** | **Sequence** | **SEQ ID NO:** |
|---|---|---|---|
| 3A2.004 (SEQ ID NO: 44) | Flag-tag | DYKDDDDK | 162 |
| | 3A2 | EVQLVESGGEQVQPGGSLKLSCSVSGLMISGHAMSWVRQVPGAGPEWVAVDNPGG KKNYIDSVAGRFTIARDNDANSLSLVMNGLRPEDTARYFCARVLSFKIYEGDXWA LEWDYRGPGTQVTVSS | 16 |
| | linker | GS | 157 |
| | VHL₁₅₂₋₂₁₃ | TLPVYTLKERCLQVVRSLVKPENYRRLDIVRSLYEDLEDHIPNVQKDLERLTQERI AHQRMGD | 20 |
| 3A2.005 (SEQ ID NO: 45) | Flag-tag | DYKDDDDK | 162 |
| | 3A2 | EVQLVESGGEQVQPGGSLKLSCSVSGLMISGHAMSWVRQVPGAGPEWVAVDNPGG KKNYIDSVAGRFTIARDNDANSLSLVMNGLRPEDTARYFCARVLSFKIYEGDXWA LEWDYRGPGTQVTVSS | 16 |
| | liner | GS | 157 |
| | SPOP₁₆₇₋₃₇₄ | SVNISGQNTMNMVKVPECRLADELGGLWENSRFTDCCLCVAGQEFQAHKAILAAR SPVFSAMFEHEMEESKKNRVEINDVEPEVFKEMMCFIYTGKAPNLDKMADDLLAA ADKYALERLKVMCEDALCSNLSVENAAEILILADLHSADQLKTQAVDFINYHASD VLETSGWKSMVVSIPHLVAEAYRSLASAQCPFLGPPRKRLKQS | 21 |
| 3A2.006 (SEQ ID NO: 46) | Flag-tag | DYKDDDDK | 162 |
| | 3A2 | EVQLVESGGEQVQPGGSLKLSCSVSGLMISGHAMSWVRQVPGAGPEWVAVDNPGG KKNYIDSVAGRFTIARDNDANSLSLVMNGLRPEDTARYFCARVLSFKIYEGDXWA LEWDYRGPGTQVTVSS | 16 |
| | linker | GS | 157 |
| | SOCS2₁₄₃₋₁₉₈ | PRNGTVHLYLTKPLYTSAPSLQHLLCRLTNKCTGAIWGLPLPTRLKDYLEEYKFQV | 22 |
| 3A2.007 (SEQ ID NO: 47) | Flag-tag | DYKDDDDK | 162 |
| | 3A2 | EVQLVESGGEQVQPGGSLKLSCSVSGLMISGHAMSWVRQVPGAGPEWVAVDNPGG KKNYIDSVAGRFTIARDNDANSLSLVMNGLRPEDTARYFCARVLSFKIYEGDXWA LEWDYRGPGTQVTVSS | 16 |
| | liner | GS | 157 |
| | CHIP₁₂₈₋₃₀₃ | RLNFGDDIPSALRIAKKKRWNSIEERRIHQESELIHSYLSRLIAAERERELEECQR NIEGDEDDSHVRAQQACIEAKHDKYMADMDELFSQYDEKRKKRDIPDYLCGKISF ELMREPCITPSGITYDRKDIEEHLQRVGHFDPVTRSPLTQEQLIPNLAMKEVIDA FISENGWVEDY | 23 |
| 3A2.010 (SEQ ID NO: 48) | Flag-tag | DYKDDDDK | 162 |
| | 3A2 | EVQLVESGGEQVQPGGSLKLSCSVSGLMISGHAMSWVRQVPGAGPEWVAVDNPGG KKNYIDSVAGRFTIARDNDANSLSLVMNGLRPEDTARYFCARVLSFKIYEGDXWA LEWDYRGPGTQVTVSS | 16 |
| | linker | GS | 157 |
| | ASB1₂₉₆₋₃₃₅ | VKWESLGPESRGRRKVDPEALQVFKEARSVPRTLLCLCRVAVRRALGKHRLHLIP P SLPLPDPIKKFLLHIE | 26 |
| 3A2. 011 (SEQ ID NO: 49) | Flag-tag | DYKDDDDK | 162 |
| | TRIM21₂₋₆₈ | ASAARLTMMWEEVTCPICLDPFVPVSIECGHSFCQECISQVKGGGGSVCPVCRQ RFLLKNLRPNRQLANMVNNLKEISQEARE | 27 |
| | linker | GS | 157 |
| | 3A2 | EVQLVESGGEQVQPGSSLKLSCSVSGLMISGHAMSWVRQVPGAGPEWVAVDNPGG KKNYIDSVAGRFTIARDNDANSLSLVMNGLRPEDTARYFCARVLSFKIYEGDKWA LEWDYRGPGTQVTVSS | 16 |
| 3A2. 012 (SEQ ID NO: 50) | Flag-tag | DYKDDDDK | 162 |
| | 3A2 | EVQLVESGGEQVQPGSSLKLSCSVSGLMISGHAMSWVRQVPGAGPEWVAVDNPGG KKNYIDSVAGRFTIARDNDANSLSLVMNGLRPEDTARYFCARVLSFKIYEGDKWA LEWDYRGPGTQVTVSS | 16 |
| | linker | GS | 157 |
| | RNF4₇₁₋₁₉₉ | DERRRPPRNARRLPQDHADSCVVSSDDEELSRDRDVYVTTHTPRNARDEGATGLR PSGTVSCPICMDGYSEIVQNGRLIVSTECGHVFCSQCLRDSLKNANTCPTCRKKI NHRRYHPIYI | 28 |
| 3A2. 018 (SEQ ID NO: 51) | Flag-tag | DYKDDDDK | 162 |
| | 3A2 | | 16 |
| | linker | GS | 157 |
| | UBE2D1₂₋₁₄₇ | ALKRIQKELSDLQRDPPAHCSAGPVGDDLFIHWQATIMGPPDSAYQGGVFFLTVHF PTDYPFKPPKIAFTTKIYHPNINSNGSICLDILRSQWSPALTVSKVLLSICSLLC DPNPDDPLVPDIAQIYKSDKEKYNRHAREWTQKYAM | 29 |
| 3A2. 019 (SEQ ID NO: 52) | Flag-tag | DYKDDDDK | 162 |
| | 3A2 | | 16 |
| | linker | GS | 157 |
| | UBE2D4₂₋₁₄₇ | ALKRIQKELTDLQRDPPAQCSAGPVGDDLFIHWQATIMGPNDSPYQGGVFFLTIHF PTDYPFKPPKIAFTTKIYHPNINSNGSICLDILRSQWSPALTVSKVLLSICSLLC DPNPDDPLVPEIAHTYKADREKYNRLAREWTQKYAM | 30 |
| 3A2. 020 (SEQ ID NO: 53) | Flag-tag | DYKDDDDK | 162 |
| | 3A2 | EVQLVESGGEQVQPGSSLKLSCSVSGLMISGHAMSWVRQVPGAGPEWVAVDNPGG KKNYIDSVAGRFTIARDNDANSLSLVMNGLRPEDTARYFCARVLSFKIYEGDKWA LEWDYRGPGTQVTVSS | 16 |
| | linker | GS | 157 |
| | ZNFR1₁₃₈₋₂₂₇ | SSHSGFKCPICSKSVASDEMEMHFIMCLSKPRLSYNDDVLTKDAGECVICLEELL QGDTIARLPCLCIYHKSCIDSWFEVNRSCPEHPAD | 31 |

### Example 6. Evaluation of SOX2 protein degradation capability of fusion proteins including S14 and E3 ubiquitin ligase fragments

The inventors of the present invention determined the SOX2 protein-specific degradation capability of the S14-E3 ubiquitin ligase fusion protein through the binding of S14 and SOX2.

Specifically, the expression vectors of 11 types of S14-E3 ubiquitin ligase fusion proteins produced by the method of Example 5.1 and the huSOX2.Myc protein expression vector were each transduced into MDA-MB-231 cells, which is a human breast cancer cell line, by using Lipofectamine 3000 (Lipofectamine 3000, Invitrogen, Cat no. L3000001). 24 hours after transduction, the cells were each washed twice with cold PBS buffer (Gibco, Cat no. 10010-023) and treated with M-PER protein extract (M-PER Mammalian Protein Extraction Reagent, Thermofisher, Cat no. 78505) including a protease inhibitor cocktail (Thermofisher, Cat no. 78440) to obtain cell extracts. Each of the cell extracts was uniformly disrupted by using a sonicator, centrifuged, and the supernatant was obtained. The protein amount of each supernatant was quantified by using a BCA kit (ThermoFisher, Cat no. 23227), and an equal amount of protein (30 ug) for each sample was electrophoresed on a 4-12% SDS PAGE gel (Invitrogen, Cat no. NW04125BOX).

The separated proteins as described above were transferred to a PVDF membrane (Invitrogen, Cat no. IB24002), and the membrane was blocked with blocking buffer [1× PBST buffer (1× PBS, 0.1% Tween 20) including 5% skim milk (BD, Cat no. 232100)] for 60 minutes. After blocking, the membrane was treated with primary antibodies and reacted at 4°C for 16 hours. In this case, the primary antibodies were anti-c-Myc antibody (SantaCruz, Cat no. sc-40, 1: 5,000), anti-FLAG antibody (Sigma, Cat no. F1804, 1: 2,500) or anti-α-tubulin antibody (GeneTex, Cat no. GTX628802, 1: 3,000), which were each diluted in blocking buffer. After the primary antibody reaction, the membrane was washed three times for 10 minutes each in 1× PBST buffer solution. Then, the membrane was treated with a secondary antibody respectively and reacted at room temperature for 1 hour. In this case, the secondary antibody used was an anti-mouse antibody (CST, Cat no. 7076) conjugated to horseradish peroxidase (HRP) diluted in blocking buffer solution. After the secondary antibody reaction, the membrane was washed three times for 10 minutes each in 1× PBST buffer solution.

The expression level of each protein was determined by using an Imager (Invitrogen, iBright, CL1500) after treating with ECL (Cytiva, Cat no. RPN2232 or Thermofisher, Cat no. 34096) solution. In addition, the expression level of the protein was quantified as the amount of SOX2 protein compared to α-tubulin and displayed as a graph (Graphpad prism software).

As a result, as shown in FIG. 4, it was confirmed that 9 out of 11 S14-E3 ubiquitin ligase fusion proteins degrade SOX2 protein. In particular, S14.010 and S14.012 showed SOX2 protein degradation capability of more than 70% compared to the control group (Mock).

### Example 7. Cancer cell growth inhibition and apoptosis induction effects of fusion proteins including S14 and E3 ubiquitin ligase fragments

### Example 7.1. Confirmation of cell growth inhibition and apoptosis induction effects by fusion proteins including S14 and E3 ubiquitin ligase fragments in human cancer cell lines

The cancer cell growth inhibition and apoptosis induction effects of S14-E3 ubiquitin ligase fusion protein were determined.

First of all, each mRNA was produced by Genscript Probio using a polynucleotide encoding S14.010 (SEQ ID NO: 137) or a polynucleotide encoding S14.012 (SEQ ID NO: 139) as a template.

The S14.010 mRNA or S14.012 mRNA produced as described above was transduced into human pancreatic cancer cell lines (BxPC3 cells) or human non-small cell lung cancer cell lines (NCI-H1703 cells, A549 cells) by using Lipofectamine MessengerMAX (Invitrogen, Cat no. LMRNA015). In this case, SOX2 siRNA (Origene, Cat no. SR321861-A) was used as a positive control, and siRNA (Santacruz, Cat no. sc-37007) was used as a control to confirm the effects of inducing cell death according to SOX2 protein expression in cancer cell lines.

In addition, apoptosis was confirmed by treating each transduced cell with Caspase-3/7 Green Dye for Apoptosis (Satorius, Cat no. 4440), and then analyzing images taken at 3-hour intervals using phase and fluorescence channels by using Incucyte (Satorius) equipment (using Incucyte software).

As a result, as shown in FIGS. 5a to 5c, it was confirmed that apoptosis increased in all three cancer cell lines in the SOX2 siRNA treatment group, S14.010 mRNA treatment group and S14.012 mRNA treatment group compared to the control group, respectively. In particular, it was confirmed that apoptosis and cell growth inhibition occurred more significantly in the S14.010 mRNA treatment group and the S14.012 mRNA treatment group, respectively.

### Example 7.2. Confirmation of cell growth inhibition effects of fusion proteins including S14 and E3 ubiquitin ligase fragments in human cancer cell lines

The growth inhibition effects of cancer cells were determined according to the concentration of S14-E3 ubiquitin ligase fusion protein.

Specifically, S14.010 mRNA and S14.012 mRNA produced by the method of Example 7.1 were each serially diluted by 1/2, and then transduced into human non-small cell lung cancer cell lines (A549 cells, NCI-H1703 cells) and human cervical cancer cell lines (SiHa cells) by using Lipofectamine-MessengerMax. The degree of cell growth for each cell was confirmed by taking images through the phase channel at 3-hour intervals using Incucyte (Satorius) equipment in the same manner as in Example 7.1, and analyzing the images using Incucyte software.

As a result, as shown in FIG. 6, the cell growth inhibition effects were confirmed for all cancer cell lines in the S14.010 mRNA treatment group and the S14.012 mRNA treatment group, respectively.

### Example 7.3. Effects of combined use of fusion proteins including S14 and E3 ubiquitin ligase fragments and anticancer drugs on cell growth inhibition and apoptosis induction in anticancer drug-resistant human cancer cell lines

It has been reported that the SOX2 protein is involved in the acquisition of anticancer drug resistance in cancer cells. The anticancer activity of the fusion protein of the present invention against anticancer drug-resistant cancer cells was determined.

### Example 7.3.1. Confirmation of combined effects of fusion proteins including S14 and E3 ubiquitin ligase fragments and tamoxifen in tamoxifen-resistant cancer cell lines

First of all, in order to confirm the correlation between the anticancer drug resistance and SOX2 protein, the expression level of SOX2 in MCF-7 cells (human breast cancer cells) and MCF7-Tam^{R} cells showing tamoxifen resistance was determined by using the same method as in Example 6. In this case, the primary antibody used was an anti-SOX2 antibody (CST, Cat no. 3579, 1:1000).

As a result, as shown in FIG. 7a, it was confirmed that SOX2 protein was expressed about 6 times more in MCF7-Tam^{R} cells compared to MCF7 cells.

Next, it was confirmed whether the fusion protein of the present invention, S14.010 or S14.012, could enhance the therapeutic effects of tamoxifen by inducing SOX2 protein degradation in MCF7-Tam^{R} cells, respectively.

Specifically, Lipofectamine-MessengerMax was used to transduce MCF7-Tam^{R} cells by serially diluting S14.010 mRNA and S14.012 mRNA produced by the method of Example 7.1 by 1/2, respectively. After transduction, each cell was treated with Caspase-3/7 Green Dye for Apoptosis (Satorius, Cat no. 4440) and cultured for additional 24 hours, followed by treatment with tamoxifen (20 uM). Apoptosis was confirmed by using Incucyte (Satorius) equipment in the same manner as in Example 7.1. The degree of cell growth and apoptosis signals at the last shooting time point were analyzed by using Incucyte software, and the results were expressed as a graph using Graphpad prism software.

As a result, as shown in FIG. 7b, it was confirmed that the effects of inducing apoptosis and inhibiting cell growth in cancer cells increased in the group treated with tamoxifen and S14.010 mRNA or S14.012 mRNA together compared to the group treated with tamoxifen alone in MCF7-Tam R cells.

### Example 7.3.2. Confirmation of combined effects of fusion proteins including S14 and E3 ubiquitin ligase fragments and doxorubicin in doxorubicin-resistant human cancer cell lines

The expression levels of SOX2 protein were compared in H69 cells (a human non-small cell lung cancer cell line) and H69-DOX^{R} cells that acquired doxorubicin resistance.

Specifically, each of the cells was washed twice with PBS buffer, and then, each cell was treated with cell lysis buffer (50 mM Tris, 10 mM EDTA, 1% SDS) including a protease inhibitor cocktail (Thermofisher, 78440) to obtain a cell extract. The cell extract was uniformly mixed by using a vortex and heated at 95°C for 10 minutes. The protein amount of the cell extract was quantified by using a BCA kit (ThermoFisher, 23227), and the protein sample was prepared by mixing with 10X sample buffer (Proteinsimple, Cat no. 042-195), 400 mM DTT solution and Fluorescent 5X Master Mix (Proteinsimple, Cat no. PS-ST01EZ), and heating at 95°C for 5 minutes.

SOX2 was detected with the Fluorescence Separation Module (Proteinsimple, Cat no. SM-FL004) by using anti-SOX2 antibody (CST, Cat no. 3579) and antibody anti-rabbit secondary detection module (Protein simple, Cat no. DM-001). The detection results were quantified by using the Protein Normalization Module (Proteinsimple, Cat no. DM-PN02). Detection was performed by using JESS (Proteinsimple) equipment, and analysis was performed by using Gaussian Fit of Compass for SW software.

As a result, as shown in FIG. 8a, it was confirmed that SOX2 protein expression increased approximately 2-fold in H69-DOX^{R} cells compared to H69 cells.

Next, it was confirmed whether the fusion protein of the present invention, S14.010 or S14.012, could enhance the therapeutic effects of doxorubicin by inducing SOX2 protein degradation in H69-DOX^{R} cells, respectively. In this case, the experiment was performed in the same manner as in Example 7.3.1, and doxorubicin was treated at a concentration of 10 uM.

As a result, as shown in FIG. 8b, it was confirmed that the effects of inducing apoptosis and inhibiting cell growth in cancer cells increased in the group treated with doxorubicin and S14.010 mRNA or S14.012 mRNA together compared to the group treated with doxorubicin alone in H69-DOX R cells.

### Example 8. Evaluation of SOX2 protein degradation capability of fusion proteins including 3A2 and E3 ubiquitin ligase fragments

### Example 8.1. Confirmation of SOX2 protein degradation capability of fusion proteins including 3A2 and E3 ubiquitin ligase fragments

The inventors of the present invention determined the SOX2 protein-specific degradation capability of the 3A2-E3 ubiquitin ligase fusion protein through the binding of 3A2 and SOX2.

The expression vectors of 10 types of 3A2-E3 ubiquitin ligase fusion proteins produced by the method of Example 5.2 and the huSOX2.V5 protein expression vector were each transduced into human kidney cell line (HEK-293 cells) by using Lipofectamine 3000 (Lipofectamine 3000, Invitrogen, Cat no. L3000001). The expression of SOX2 protein in the transduced cells was determined by the same method as in Example 6. In this case, anti-V5 antibody (Invitrogen, Cat no. 46-1157), anti-Flag antibody (Sigma, Cat no. F1804), Fluorescence Separation Module (Proteinsimple, Cat no. SM-FL004) and anti-mouse-HRP antibody (Proteinsimple, Cat no. 042-206) were additionally used.

As a result, as shown in FIG. 9, it was confirmed that 6 out of a total of 10 3A2-E3 ubiquitin ligase fusion proteins degraded SOX2 protein. In particular, the 3A2.005 treatment group, the 3A2.007 treatment group, the 3A2.011 treatment group and the 3A2.012 treatment group each exhibited a SOX2 degradation capability of approximately 70% or more compared to the control group (Mock).

### Example 8.2. Evaluation of SOX2 protein degradation capability according to concentration of fusion proteins including 3A2 and E3 ubiquitin ligase fragments in human cancer cell lines

The inventors of the present invention determined the effects of intracellular SOX2 protein degradation on the concentration of fusion proteins including 3A2 and E3 ubiquitin ligase fragments in human cancer cell lines.

First of all, the polynucleotide encoding 3A2.005 (SEQ ID NO: 141) or the polynucleotide encoding 3A2.012 (SEQ ID NO: 146) was cloned into the Linearized pIVT vector (TAKARA, Cat no. 6143) according to the manufacturer's method by using the TAKARA Cloning Kit for mRNA Template (TAKARA, Cat no. 6143). By using the vector as a template, mRNA encoding 3A2.005 and 3A2.012, respectively, were prepared by using the Takara IVTpro^{™} T7 mRNA Synthesis Kit (TAKARA, Cat no. 6144), CleanCap Reagent AG (TriLink, Cat no. N7113-10) and N1-Methyl-Pseudouridine-5'-Triphosphate (TriLink, Cat no. N1081-10).

The 3A2.005 mRNA and 3A2.012 mRNA were transduced into human non-small cell lung cancer cell lines (NSCLC) (A549 cells, NCI-H1703 cells, HCC827 cells, SK-MES-1 cells, SW900 cells) and human hepatoma cell lines (HCC) (Hep3B cells) by using Lipofectamine MessengerMAX (Invitrogen, Cat no. LMRNA015), respectively. The expression of SOX2 protein in the transduced cells was determined by the same method as in Example 7.3.2.

As a result, as shown in FIG. 10, it was confirmed that the 3A2.005 mRNA treatment group or the 3A2.012 mRNA treatment group increased SOX2 degradation in a treatment concentration-dependent manner in all cancer cell lines. The concentration at which the maximum degradation of SOX2 protein occurred (DC₅₀, nM) for each cancer cell line is listed in Table 9.

**[Table 9]**

| Cancer cell line | | DC₅₀ (nM) | |
|---|---|---|---|
| | | 3A2.005 | 3A2.012 |
| NSCLC | A549 | 0.04 | 0.02 |
| | NCI-H1703 | 0.03 | 0.05 |
| | HCC827 | 0.01 | 0.05 |
| | SK-MES-1 | 0.03 | 0.13 |
| | SW900 | 0.12 | 0.57 |
| HCC | Hep3B | 0.05 | 0.03 |

### Example 8.3. Confirmation of ubiquitin-proteasome-dependent SOX2 protein degradation by fusion proteins including 3A2 and E3 ubiquitin ligase fragments

The inventors of the present invention determined that the degradation of SOX2 protein induced by the 3A2-E3 ubiquitin ligase fusion protein occurs via the intracellular ubiquitin-proteasome system (UPS).

Specifically, the 3A2.005 expression vector, 3A2.012 expression vector and huSOX2.V5 expression vector produced by the method of Example 5.2 were transduced into human kidney cell line (HEK-293 cells) by using Lipofectamine 3000 (Lipofectamine 3000, Invitrogen, Cat no. L3000001). 18 hours after transduction, the cells were treated with MLN7243, which is an ubiquitin E1 activating enzyme inhibitor, or MG132, which is a proteasome inhibitor, at concentrations of 1 uM and 20 uM, respectively, and incubated for additional 6 hours. In this case, DMSO was treated as a control group. The amount of SOX2 protein expression in the cells treated as described above was determined by the same method as Example 7.3.2. In this case, anti-V5 antibody (Invitrogen, Cat no. 46-1157) and anti-Flag antibody (Sigma, cat no. F1804) were used.

As a result, as shown in FIG. 11, compared to the control group, the SOX2 protein degradation effect was significantly reduced by the expression of 3A2.005 or 3A2.012 in the MLN7243 treatment group and the MG132 treatment group, respectively. Therefore, it was confirmed that the degradation of SOX2 protein by the expression of the fusion proteins including the 3A2 and E3 ubiquitin ligase fragments was induced in a ubiquitin-proteasome dependent manner.

### Example 9. Cell growth inhibition effect of fusion proteins including 3A2 and E3 ubiquitin ligase fragments on cancer cells

### Example 9.1. Comparison of cell growth inhibition effects of fusion proteins including SOX2 siRNA and 3A2 and E3 ubiquitin ligase fragments on cancer cells

SOX2 siRNA (Origene, Cat no. SR321861-A) and 3A2.005 mRNA or 3A2.012 mRNA were transduced into human non-small cell lung cancer cell lines (A549 cells) by using Lipofectamine RNAiMAX (Lipofectamine RNAiMAX, Invitrogen, Cat no. 13778150) and Lipofectamine MessengerMAX (Invitrogen, Cat no. LMRNA015), and the degree of cell growth inhibition was confirmed. In this case, the 3A2.005 mRNA and 3A2.012 mRNA were produced and used in the same manner as in Example 8.2. In addition, the detection of the intracellular SOX2 protein expression level and the degree of cancer cell growth inhibition were determined in the same manner as in Example 7.3.2 and Example 7.2.

As a result, as shown in FIG. 12, it was confirmed that the SOX2 protein degradation and cancer cell growth inhibition effects increased in the 3A2.005 mRNA treatment group and the 3A2.012 mRNA treatment group, respectively, compared to the SOX2 siRNA treatment group.

### Example 9.2. Confirmation of efficacy according to concentration of fusion proteins including 3A2 and E3 ubiquitin ligase in human cancer cell lines

The growth inhibition effects of cancer cells according to the concentration of 3A2-E3 ubiquitin ligase fusion protein treatment in human cancer cell lines were determined.

Specifically, 3A2.005 mRNA and 3A2.012 mRNA were serially diluted and transduced into human non-small cell lung cancer cell lines (A549 cells, NCI-H1703 cells, Calu-1 cells, HCC15 cells, SW900 cells, SK-MES-1 cells), human pancreatic cancer cell lines (BxPC3 cells, CFPAC-1 cells), human cervical cancer (SiHa cells) or human hepatoma cell lines (HepG2 cells) by using Lipofectamine-MessengerMax. The degree of cell growth of the transduced cells was captured as images by using the phase channel at 3-hour intervals with Incucyte (Satorius) equipment, and then analyzed by using Incucyte software at the last capture time point. The 3A2.005 mRNA and 3A2.012 mRNA were produced and used in the same manner as in Example 8.2.

As a result, as shown in FIGS. 13a and 13b, the 3A2.005 mRNA treatment group and the 3A2.012 mRNA treatment group showed cell growth inhibition effects on all cancer cell lines. The GI₅₀ (nM) values, which are the concentrations that inhibited 50% of the maximum cell proliferation of the cancer cell lines, are shown in Table 10.

**[Table 10]**

| Cancer cell line | | GI₅₀ (nM) | |
|---|---|---|---|
| | | 3A2.005 | 3A2.012 |
| NSCLC | A549 | 0.43 | 1.16 |
| | NCI-H1703 | 0.11 | 0.15 |
| | Calu-1 | 0.51 | 1.26 |
| | HCC15 | 0.33 | 0.84 |
| | SW900 | 0.14 | 0.45 |
| | SK-MES-1 | 0.45 | 0.57 |
| Pancreatic cancer | CFPAC-1 | <0.01 | 0.94 |
| | BxPC3 | 0.45 | 0.48 |
| Cervical cancer | SiHa | 0.19 | 0.25 |
| HCC | HepG2 | 0.43 | 0.93 |

### Example 9.3. Effects of combined use of fusion proteins including 3A2 and E3 ubiquitin ligase fragments and anticancer drugs on cell growth inhibition and apoptosis induction in anticancer drug-resistant human cancer cell lines

The combined effects were determined in the same manner as in Example 7.2 by transducing tamoxifen or doxorubicin, and 3A2.005 mRNA or 3A2.012 mRNA at 1 nM each in tamoxifen-resistant breast cancer cell line MCF7-Tam^{R} cells and doxorubicin-resistant lung cancer cell line H69-Dox R cells. In this case, tamoxifen was treated at each concentration by serially diluting 2/3 from 100 uM, and doxorubicin was treated at each concentration by serially diluting 1/2 from 150 uM.

As a result, as shown in FIG. 14, the apoptosis induction and cell growth inhibition effects were shown to be higher in the tamoxifen and 3A2.005 mRNA or 3A2.012 mRNA combination treatment group than in the tamoxifen alone treatment group in MCF7-Tam^{R} cells. In addition, in H69-Dox^{R} cells, the apoptosis induction and cell growth inhibition effects were shown to be higher in the doxorubicin and 3A2.005 mRNA or 3A2.012 mRNA combination treatment group than in the doxorubicin alone treatment group.

### Example 10. Evaluation of tumor growth inhibitory effects of fusion proteins including S14 and E3 ubiquitin ligase fragments in human cancer cell line transplantation tumor mouse model

In order to confirm the tumor suppression efficacy of the S14-E3 ubiquitin ligase fusion protein, a mouse tumor model was created by transplanting a non-small cell lung cancer cell line (A549 cells) or a pancreatic cancer cell line (BxPC3 cells).

Specifically, 5.0 × 10⁶ cells (A549 cells) or 1.0 × 10⁷ cells (BxPC3 cells) were subcutaneously implanted into 6- to 8-week-old female M-NSG mice (Shanghai Model Organisms Center, Inc.). Then, when the tumor size of each individual reached approximately 150 mm³, they were randomly divided into groups of 5 per group. Thereafter, lipid nanoparticles including S14.012 mRNA were intravenously administered once a week for a total of 3 times at a concentration of 2 mg/kg. In this case, PBS was intravenously administered to the control group.

Tumor volume was calculated by using <Mathematical Formula 1> below based on the values measured twice a week. The body weight of each individual was also measured at this time.$Tumor volume \left({mm}^{3}\right) = 0.5 \times \left(long axis\right) \times {\left(short axis\right)}^{2}$

As a result, as shown in FIG. 15, the S14.012 mRNA administration group showed a tumor growth inhibitory effect in both mouse tumor models implanted with A549 cells or BxPC3 cells. In this case, no significant change in body weight was observed in the S14.012 mRNA administration group compared to the control group.

### Example 11. Confirmation of the cancer cell growth inhibition and apoptosis induction effects of fusion proteins including 3A2 and E3 ubiquitin ligase fragments in human non-small cell lung cancer cell lines

3A2.005 mRNA or 3A2.012 mRNA was transduced into human non-small cell lung cancer cell lines (A549 cells) by using Lipofectamine MessengerMAX (Invitrogen, Cat no. LMRNA015). In this case, SOX2 siRNA (Origene, Cat no. SR321861-A) and doxorubicin were used as positive controls, and control siRNA (Santacruz, Cat no. sc-37007) was used as a control group to determine the effects of inducing cell death according to the regulation of SOX2 protein expression in cancer cell lines. The 3A2.005 mRNA or 3A2.012 mRNA was produced and used in the same manner as in Example 8.2.

In addition, apoptosis was confirmed by treating each transduced cell with Caspase-3/7 Green Dye for Apoptosis (Satorius, Cat no. 4440), and then analyzing images taken at 3-hour intervals by using phase and fluorescence channels using Incucyte (Satorius) equipment (using Incucyte software).

As a result, as shown in FIG. 16, it was confirmed that cell growth was inhibited and cell death increased in the SOX2 siRNA treatment group, doxorubicin, 3A2.005 mRNA treatment group and 3A2.012 mRNA treatment group compared to the control group in the cancer cell lines. In particular, cell growth inhibition and cell death in the 3A2.005 mRNA treatment group and the 3A2.012 mRNA treatment group were confirmed at a level similar to that of doxorubicin, which was the positive control group.

### Example 12. Expression of fusion proteins including 3A2 and E3 ubiquitin ligase fragments and evaluation of persistence of SOX2 protein degradation

The expression of 3A2-E3 ubiquitin ligase fusion protein in cells and the duration of the fusion protein's capability to degrade SOX2 proteins were confirmed.

Specifically, the expression vectors for the 3A2.005 fusion protein or the 3A2.012 fusion protein produced by the method of Example 5.2 above were each transduced with the huSOX2.V5 protein expression vector into human kidney cell lines (HEK-293 cells) by using Lipofectamine 3000 (Invitrogen, Cat no. L3000001). Then, cell extracts were obtained at 24-hour intervals from 72 hours to 168 hours. The amount of SOX2 protein in the cell extracts was determined by the same method as in the above Example 7.3.2. In this case, SOX2 was detected by using the primary antibody anti-V5 antibody (Invitrogen, Cat no. 46-1157), and the 3A2-E3 ubiquitin ligase fusion protein was detected by using anti-Flag antibody (Sigma, cat no. F1804). The secondary antibody used was anti-mouse-HRP antibody (Proteinsimple, Cat no. 042-206).

As a result, as shown in FIG. 17, it was confirmed that the expression of the 3A2.005 and 3A2.012 fusion proteins and the SOX2 protein degradation effects of the fusion proteins continued from 72 to 168 hours.

### Example 13. Comparison of SOX2 protein degradation capability according to treatment concentration of fusion proteins including 3A2 and E3 ubiquitin ligase fragments and SOX2 siRNA

SOX2 siRNA (Origene, Cat no. SR321861-A) or 3A2.012 mRNA was transduced into human non-small cell lung cancer cell line (SW900 cells) by using Lipofectamine-RNAiMAX (Lipofectamine RNAiMAX, Invitrogen, Cat no. 13778150) or Lipofectamine-MessengerMax, respectively, and the amount of intracellular SOX2 protein was detected in the same manner as in Example 7.3.2. The results were expressed as a graph by using Graphpad prism software. 3A2.012 mRNA was prepared and used in the same manner as in Example 8.2.

As a result, as shown in FIG. 18, it was confirmed that the SOX2 protein degradation capability significantly increased in a concentration-dependent manner in the 3A2.012 mRNA treatment group compared to the SOX2 siRNA treatment group.

### Example 14. Confirmation of SOX2 protein degradation capability according to treatment concentrations of fusion proteins including 3A2 and E3 ubiquitin ligase fragments in human cancer cell lines

The inventors of the present invention determined the intracellular SOX2 protein degradation capability according to the concentration of 3A2-E3 ubiquitin ligase fusion protein in human cancer cell lines.

3A2.005 mRNA and 3A2.007 mRNA were serially diluted at each concentration by using Lipofectamine-MessengerMax and transduced into human non-small cell lung cancer cell lines (NSCLC) (A549 cells, SW900 cells). In this case, 3A2.007 mRNA was produced and used in the same manner as in Example 8.2 using a polynucleotide (SEQ ID NO: 143) encoding 3A2.007. In addition, 3A2.005 mRNA was also produced and used in the same manner as in Example 8.2. After 24 hours, each cell extract was obtained, and the amount of SOX2 protein was determined in the same manner as in Example 7.3.2.

As a result, as shown in FIG. 19, it was confirmed that the 3A2.005 mRNA treatment group and the 3A2.007 mRNA treatment group increased SOX2 degradation in a concentration-dependent manner in all cancer cell lines, respectively. The concentration at which the maximum degradation of SOX2 protein occurred (DC₅₀, nM) for each cancer cell line is shown in Table 11.

**[Table 11]**

| Cancer cell line | | DC₅₀ (nM) | |
|---|---|---|---|
| | | 3A2.005 | 3A2.007 |
| NSCLC | A549 | 0.10 | 0.03 |
| | SW900 | 0.29 | 0.18 |

### Example 15. Evaluation of cell growth inhibition efficacy according to concentration of fusion proteins including 3A2 and E3 ubiquitin ligase fragments in human cancer cell lines

The cell growth inhibition efficacy of 3A2-E3 ubiquitin ligase fusion protein was confirmed according to the concentration in human cancer cell lines.

3A2.005 mRNA or 3A2.007 mRNA was serially diluted at each concentration and transduced into human non-small cell lung cancer cell lines (NSCLC) (A549 cells, SW900 cells) by using Lipofectamine-MessengerMax, respectively. Images were taken at 3-hour intervals by using the phase channel through Incucyte (Satorius) equipment, and the degree of cell growth inhibition was analyzed (using Incucyte software). The cell growth inhibition results after 72 hours were expressed as a graph by using Graphpad prism software. 3A2.005 mRNA and 3A2.007 mRNA were produced and used in the same manner as in Example 8.2.

As a result, as shown in FIG. 20, the 3A2.005 mRNA treatment group and the 3A2.007 mRNA treatment group each exhibited a cell growth inhibition effect in a concentration-dependent manner of mRNA treatment in all cancer cell lines. The maximum cell proliferation inhibitory concentration of 50% (GI₅₀, nM) of each cancer cell line is shown in Table 12.

**[Table 12]**

| Cancer cell line | | GI₅₀ (nM) | |
|---|---|---|---|
| | | 3A2.005 | 3A2.007 |
| NSCLC | A549 | 1.58 | 0.37 |
| | SW900 | 0.34 | 0.21 |

### Example 16. Confirmation of specific SOX2 protein degradation and cell growth inhibition effects of fusion proteins including 3A2 and E3 ubiquitin ligase fragments in human non-small cell lung cancer cell lines

The SOX2 protein degradation and cell growth inhibition effects of 3A2 single domain antibody, E3 ubiquitin ligase protein fragment (alone) or 3A2-E3 ubiquitin ligase fusion protein were compared in human non-small cell lung cancer cell lines (A549 cells, SW900 cells).
mRNA encoding 3A2 single domain antibody, E3 ubiquitin ligase protein (alone) (3A2.005 E3 only, 3A2.007 E3 only) or 3A2-E3 ubiquitin ligase fusion protein (3A2.005, 3A2.007), respectively, was transduced into human non-small cell lung cancer cell lines (A549 cells, SW900 cells) by using Lipofectamine-MessengerMax. In this case, A549 cells were treated with 0.3 nM mRNA, and SW900 cells were treated with 1 nM mRNA, respectively. The mRNA of the 3A2 single domain antibody and the mRNA of the E3 ubiquitin ligase protein fragment were produced by using the polynucleotides of SEQ ID NO: 112 (3A2), SEQ ID NO: 117 (SPOP₁₆₇₋₃₇₄) and SEQ ID NO: 119 (CHIP₁₂₈₋₃₀₃), respectively, by the method of Example 8.2. In addition, 3A2.005 mRNA and 3A2.007 mRNA were also produced by using the same method as Example 8.2.

The expression of SOX2 protein in transduced cells and the inhibitory effect on cancer cell growth were determined by the same methods as in Example 7.3.2 and Example 7.2, respectively. For the detection of single domain antibodies, E3 ubiquitin ligase fragments and 3A2-E3 ubiquitin ligase fusion proteins, anti-FLAG antibody (Sigma, Cat no. F1804) was used as the primary antibody, and anti-mouse-HRP antibody (Proteinsimple, Cat no. 042-206) was used as the secondary antibody.

As a result, as shown in FIGS. 21a and 21b, in the single domain antibody (binder only) mRNA treatment group and the E3 ubiquitin ligase protein fragment (only) (E3 only) mRNA treatment group, the degradation of SOX2 protein (FIG. 21a) and the inhibition of cancer cell growth (FIG. 21b) were not observed. In contrast, in both the 3A2.005 mRNA treatment group and the 3A2.007 mRNA treatment group, the degradation of SOX2 protein and the inhibition of cancer cell growth were observed.

### Example 17. Confirmation of SOX2 protein degradation capability of fusion proteins including 3A2 and E3 ubiquitin ligase fragments in human cancer cell line transplantation tumor mouse model

The tumor suppression efficacy of the 3A2-E3 ubiquitin ligase fusion protein was determined by using a mouse tumor model transplanted with a non-small cell lung cancer cell line (A549 cells). In this case, the mouse tumor model was produced by using the same method as in Example 10.

When the tumor size of each individual mouse model above reached about 150-200 mm³, each mouse was randomly divided into groups of 3 mice. Then, lipid nanoparticles including 3A2.012 mRNA were administered intravenously once at a concentration of 2 mg/kg, and tumor tissues were extracted from the mice 16 hours later. In this case, PBS was administered intravenously to the control group. 3A2.012 mRNA was prepared and used by the method of Example 8.2.

The extracted tumor tissue was treated with cell lysis buffer and finely ground by using a TissueLyser. Then, the ground substance was sonicated (10 seconds, 10 times) and centrifuged (15,000 rpm, 10 minutes) to obtain a tumor tissue extract. The amount of SOX2 protein in the tumor tissue was determined by using the same method as in Example 6. In this case, the primary antibody used was anti-SOX2 antibody (CST, Cat no. 3579) or anti-GAPDH antibody (Abcam, Cat no. 8245).

As a result, as shown in FIG. 22, it was confirmed that SOX2 protein was reduced in the tumor tissues of all individuals (3 mice) in the 3A2.012 mRNA treatment group compared to the control group.

### Example 18. Evaluation of SOX2 protein degradation capability according to treatment time of fusion proteins including 3A2 and E3 ubiquitin ligase fragments in human non-small cell lung cancer cell lines

After expression of the 3A2-E3 ubiquitin ligase fusion protein in human non-small cell lung cancer cell lines, the extent of SOX2 protein degradation over time was determined.

Specifically, 3A2.005 mRNA and 3A2.012 mRNA were transduced at a concentration of 2 nM into human non-small cell lung cancer cell line (SW900 cells) by using Lipofectamine-MessengerMax, respectively. The transduced cells were cultured for each hour (4, 8, and 24 hours), and cell extracts were obtained to determine the expression of SOX2 protein in the same manner as in Example 7.3.2. The results were expressed as a graph by using Graphpad prism software. 3A2.005 mRNA and 3A2.012 mRNA were produced and used in the same manner as in Example 8.2.

As a result, as shown in FIG. 23, SOX2 protein degradation was observed 4 hours after treatment with 3A2.005 mRNA or 3A2.012 mRNA, and it was confirmed that the degree of SOX2 protein degradation increased over time.

### Example 19. Expression of fusion proteins including 3A2 and E3 ubiquitin ligase fragments and evaluation of persistence of SOX2 protein degradation in human non-small cell lung cancer cell lines

After treating human non-small cell lung cancer cell lines with mRNA encoding a 3A2-E3 ubiquitin ligase fusion protein, the duration of the fusion protein's capability to degrade SOX2 protein was determined.

Specifically, 3A2.005 mRNA, 3A2.007 mRNA, and 3A2.012 mRNA were transduced at a concentration of 1 nM into human non-small cell lung cancer cell line (SW900 cells) by using Lipofectamine-MessengerMax, respectively. The transduced cells were cultured for each hour (0, 4, 8, 24, 48, 72, 96, 120, 144, and 168 hours) to obtain cell extracts. The intracellular SOX2 protein in each treatment group was confirmed by using the same method as in Example 6, and represented as a graph by using Graphpad prism software. 3A2.005 mRNA, 3A2.007 mRNA, and 3A2.012 mRNA were produced and used by using the same method as in Example 8.2.

As a result, as shown in FIGS. 24a and 24b, when 3A2.005, 3A2.007 and 3A2.012 were treated, the SOX2 degradation capability was highest 24 hours after treatment, and the degradation efficacy was confirmed to last until the 7th day. Inversely, the expression level of the fusion protein was highest at 24 or 48 hours and decreased thereafter.

### Example 20. Effects of inducing cancer cell growth inhibition by combined use of fusion proteins including 3A2 and E3 ubiquitin ligase fragments and doxorubicin in doxorubicin-resistant lung cancer cell lines

The combined effect of 3A2-E3 ubiquitin ligase fusion protein and standard-of-care drugs was investigated to determine whether the fusion protein could enhance the therapeutic effect of standard-of-care drugs by inducing SOX2 protein degradation in human non-small cell lung cancer cell line H69 cells and doxorubicin-resistant lung cancer cell line H69-DoxR cells.

Specifically, 3A2.005 mRNA, 3A2.007 mRNA and 3A2.012 mRNA were transduced into H69 cells or H69-Dox^{R} cells at a concentration of 5 nM each by using Lipofectamine-MessengerMax. Simultaneously with transduction, H69-Dox^{R} cells were treated with doxorubicin at a 1/4 serial dilution starting from 50 uM, and H69 cells were treated with doxorubicin at a 1/4 serial dilution starting from 30 uM, and then cultured. In order to determine the degree of cell growth inhibition, at the end of the culture, the Cell Counting Kit-8 reagent (Dojindo, CK04) was treated and reacted, and then, the absorbance was measured by using a microplate reader (BioTek, Synergy HTX). The results were expressed as a graph by using Graphpad prism software. The clinical dose indicated in the graph was converted to a cell area basis and indicated in the corresponding concentration range. 3A2.005 mRNA, 3A2.007 mRNA and 3A2.012 mRNA were produced and used in the same manner as in Example 8.2.

As a result, as shown in FIGS. 25 and 26, H69-Dox^{R} cells acquired doxorubicin resistance, and it was confirmed that when doxorubicin and 3A2.005 mRNA, 3A2.007 mRNA or 3A2.012 mRNA were combined and treated in a concentration range where cancer cell growth inhibition did not occur (single treatment dose range: 8.3 uM to 10.3 uM; combination treatment dose range: 5.5 uM to 10.3 uM), cancer cell growth was inhibited.

Additionally, in H69 cells without resistance, it was confirmed that the growth of cancer cells was further inhibited when doxorubicin and 3A2.005 mRNA, 3A2.007 mRNA or 3A2.012 mRNA were combined, compared to when doxorubicin was treated alone at the lowest concentration of the clinical dose range, 5.5 uM or less.

Through the above results, it was confirmed that co-administration of the fusion protein of the present invention and doxorubicin may improve (increase) the efficacy of doxorubicin.

### Example 21. Effects of inducing cancer cell growth inhibition by combined use of fusion proteins including 3A2 and E3 ubiquitin ligase fragments and tamoxifen in tamoxifen-resistant breast cancer cell lines

The inventors of the present invention determined the combined effects of 3A2-E3 ubiquitin ligase fusion protein and standard-of-care drugs by inducing SOX2 protein degradation in human breast cancer cell line MCF7 and tamoxifen-resistant breast cancer cell line MCF7-Tam^{R} cells.

Specifically, 3A2.005 mRNA, 3A2.007 mRNA and 3A2.012 mRNA were transduced into MCF7 cells or MCF7-Tam^{R} cells at a concentration of 5 nM each by using Lipofectamine-MessengerMax. After transduction, the cells were cultured for 24 hours, and tamoxifen was diluted from 27 uM to 3 uM in 3 uM intervals and treated to each cell, followed by additional culture for 48 hours. The degree of cell growth inhibition was measured in the same manner as in Example 20. The indicated clinical dose was converted to a cell area standard and indicated in the corresponding concentration range. 3A2.005 mRNA, 3A2.007 mRNA and 3A2.012 mRNA were produced and used in the same manner as in Example 8.2.

As a result, as shown in FIG. 25 and FIG. 27, it was confirmed that cell growth was inhibited in MCF7-Tam^{R} cells when tamoxifen and 3A2.005 mRNA, 3A2.007 mRNA or 3A2.012 mRNA were co-treated in a concentration range (2.01 uM to 4.08 uM) where cell growth inhibition due to tamoxifen resistance did not occur.

In addition, it was confirmed that cell growth was inhibited by more than 50% in the combination treatment group of tamoxifen and 3A2.005 mRNA, 3A2.007 mRNA or 3A2.012 mRNA compared to the tamoxifen alone treatment group in a clinical dose concentration range (2.01 uM to 4.08 uM) in non-resistant MCF7 cells.

Through the above results, it was confirmed that combined administration of the fusion protein of the present invention and tamoxifen may improve (increase) the efficacy of tamoxifen.

## Claims

1. An anti-SOX2 single domain antibody or an antigen-binding fragment thereof, comprising a heavy chain variable region comprising HCDR1 comprising any one amino acid sequence selected from the group consisting of SEQ ID NO: 56, SEQ ID NO: 63, SEQ ID NO: 69, SEQ ID NO: 73, SEQ ID NO: 78, SEQ ID NO: 82, SEQ ID NO: 87 and SEQ ID NO: 94; HCDR2 comprising any one amino acid sequence selected from the group consisting of SEQ ID NO: 58, SEQ ID NO: 65, SEQ ID NO: 71, SEQ ID NO: 72, SEQ ID NO: 74, SEQ ID NO: 75, SEQ ID NO: 76, SEQ ID NO: 79, SEQ ID NO: 84 and SEQ ID NO: 89; and HCDR3 comprising any one amino acid sequence selected from the group consisting of SEQ ID NO: 60, SEQ ID NO: 67, SEQ ID NO: 81, SEQ ID NO: 85, SEQ ID NO: 91 and SEQ ID NO: 96.

2. The anti-SOX2 single domain antibody or antigen-binding fragment thereof according to claim 1, wherein the antigen-binding fragment comprises a heavy chain variable region comprising HCDR1 comprising an amino acid sequence of SEQ ID NO: 56, HCDR2 comprising an amino acid sequence of SEQ ID NO: 58; and HCDR3 comprising an amino acid sequence of SEQ ID NO: 60.

3. The anti-SOX2 single domain antibody or antigen-binding fragment thereof according to claim 2, wherein a heavy chain variable region of the antibody comprises an amino acid sequence of SEQ ID NO: 5.

4. The anti-SOX2 single domain antibody or antigen-binding fragment thereof according to claim 1, wherein the antibody comprises a heavy chain variable region comprising HCDR1 comprising an amino acid sequence of SEQ ID NO: 63, HCDR2 comprising an amino acid sequence of SEQ ID NO: 65; and HCDR3 comprising an amino acid sequence of SEQ ID NO: 67.

5. The anti-SOX2 single domain antibody or antigen-binding fragment thereof according to claim 4, wherein a heavy chain variable region of the antibody comprises an amino acid sequence of SEQ ID NO: 6.

6. The anti-SOX2 single domain antibody or antigen-binding fragment thereof according to claim 1, wherein the antibody comprises a heavy chain variable region comprising HCDR1 comprising an amino acid sequence of SEQ ID NO: 69, HCDR2 comprising an amino acid sequence of SEQ ID NO: 71; and HCDR3 comprising an amino acid sequence of SEQ ID NO: 67.

7. The anti-SOX2 single domain antibody or antigen-binding fragment thereof according to claim 6, wherein a heavy chain variable region of the antibody comprises an amino acid sequence of SEQ ID NO: 7.

8. The anti-SOX2 single domain antibody or antigen-binding fragment thereof according to claim 1, wherein the antibody comprises a heavy chain variable region comprising HCDR1 comprising an amino acid sequence of SEQ ID NO: 63, HCDR2 comprising an amino acid sequence of SEQ ID NO: 72; and HCDR3 comprising an amino acid sequence of SEQ ID NO: 67.

9. The anti-SOX2 single domain antibody or antigen-binding fragment thereof according to claim 8, wherein a heavy chain variable region of the antibody comprises an amino acid sequence of SEQ ID NO: 8.

10. The anti-SOX2 single domain antibody or antigen-binding fragment thereof according to claim 1, wherein the antibody comprises a heavy chain variable region comprising HCDR1 comprising an amino acid sequence of SEQ ID NO: 73, HCDR2 comprising an amino acid sequence of SEQ ID NO: 74; and HCDR3 comprising an amino acid sequence of SEQ ID NO: 67.

11. The anti-SOX2 single domain antibody or antigen-binding fragment thereof according to claim 10, wherein a heavy chain variable region of the antibody comprises an amino acid sequence of SEQ ID NO: 9.

12. The anti-SOX2 single domain antibody or antigen-binding fragment thereof according to claim 1, wherein the antibody comprises a heavy chain variable region comprising HCDR1 comprising an amino acid sequence of SEQ ID NO: 63, HCDR2 comprising an amino acid sequence of SEQ ID NO: 75; and HCDR3 comprising an amino acid sequence of SEQ ID NO: 67.

13. The anti-SOX2 single domain antibody or antigen-binding fragment thereof according to claim 12, wherein a heavy chain variable region of the antibody comprises an amino acid sequence of SEQ ID NO: 10.

14. The anti-SOX2 single domain antibody or antigen-binding fragment thereof according to claim 12, wherein a heavy chain variable region of the antibody comprises an amino acid sequence of SEQ ID NO: 11.

15. The anti-SOX2 single domain antibody or antigen-binding fragment thereof according to claim 1, wherein the antibody comprises a heavy chain variable region comprising HCDR1 comprising an amino acid sequence of SEQ ID NO: 63, HCDR2 comprising an amino acid sequence of SEQ ID NO: 76; and HCDR3 comprising an amino acid sequence of SEQ ID NO: 67.

16. The anti-SOX2 single domain antibody or antigen-binding fragment thereof according to claim 15, wherein a heavy chain variable region of the antibody comprises an amino acid sequence of SEQ ID NO: 12.

17. The anti-SOX2 single domain antibody or antigen-binding fragment thereof according to claim 1, wherein the antibody comprises a heavy chain variable region comprising HCDR1 comprising an amino acid sequence of SEQ ID NO: 82, HCDR2 comprising an amino acid sequence of SEQ ID NO: 84; and HCDR3 comprising an amino acid sequence of SEQ ID NO: 85.

18. The anti-SOX2 single domain antibody or antigen-binding fragment thereof according to claim 17, wherein a heavy chain variable region of the antibody comprises an amino acid sequence of SEQ ID NO: 13.

19. The anti-SOX2 single domain antibody or antigen-binding fragment thereof according to claim 1, wherein the antibody comprises a heavy chain variable region comprising HCDR1 comprising an amino acid sequence of SEQ ID NO: 78, HCDR2 comprising an amino acid sequence of SEQ ID NO: 79; and HCDR3 comprising an amino acid sequence of SEQ ID NO: 81.

20. The anti-SOX2 single domain antibody or antigen-binding fragment thereof according to claim 19, wherein a heavy chain variable region of the antibody comprises an amino acid sequence of SEQ ID NO: 14.

21. The anti-SOX2 single domain antibody or antigen-binding fragment thereof according to claim 1, wherein the antibody comprises a heavy chain variable region comprising HCDR1 comprising an amino acid sequence of SEQ ID NO: 87, HCDR2 comprising an amino acid sequence of SEQ ID NO: 89; and HCDR3 comprising an amino acid sequence of SEQ ID NO: 91.

22. The anti-SOX2 single domain antibody or antigen-binding fragment thereof according to claim 21, wherein a heavy chain variable region of the antibody comprises an amino acid sequence of SEQ ID NO: 15.

23. The anti-SOX2 single domain antibody or antigen-binding fragment thereof according to claim 1, wherein the antibody comprises a heavy chain variable region comprising HCDR1 comprising an amino acid sequence of SEQ ID NO: 94, HCDR2 comprising an amino acid sequence of SEQ ID NO: 89; and HCDR3 comprising an amino acid sequence of SEQ ID NO: 91.

24. The anti-SOX2 single domain antibody or antigen-binding fragment thereof according to claim 23, wherein a heavy chain variable region of the antibody comprises an amino acid sequence of SEQ ID NO: 16.

25. The anti-SOX2 single domain antibody or antigen-binding fragment thereof according to claim 1, wherein the antibody comprises a heavy chain variable region comprising HCDR1 comprising an amino acid sequence of SEQ ID NO: 94, HCDR2 comprising an amino acid sequence of SEQ ID NO: 89; and HCDR3 comprising an amino acid sequence of SEQ ID NO: 96.

26. The anti-SOX2 single domain antibody or antigen-binding fragment thereof according to claim 25, wherein a heavy chain variable region of the antibody comprises an amino acid sequence of SEQ ID NO: 17.

27. A polynucleotide, encoding the anti-SOX2 single domain antibody or antigen-binding fragment thereof according to any one of claims 1 to 26.

28. An expression vector, loaded with the polynucleotide according to claim 27.

29. A transformed cell, into which the expression vector according to claim 28 is introduced.

30. A method for producing an anti-SOX2 single domain antibody or an antigen-binding fragment thereof, the method comprising:
i) culturing the transformed cell according to claim 29; and
ii) obtaining an anti-SOX2 single domain antibody or an antigen-binding fragment thereof from the culture medium.

31. A fusion protein, comprising the anti-SOX2 single domain antibody or antigen-binding fragment thereof according to claim 1; and a fragment of E3 ubiquitin ligase or a variant thereof.

32. The fusion protein according to claim 31, wherein the fragment of E3 ubiquitin ligase or variant thereof is any one selected from the group consisting of βTrCP (Beta-transducin repeat-containing protein), SKP2 (S-phase kinase-associated protein 2), VHL (von Hippel-Lindau), SPOP (speckle-type BTB-POZ), SOCS2 (suppressor of cytokine signaling 2), CHIP (carboxy terminus of Hsp70-binding protein), DDB2 (damage DNA binding protein 2), CRBN (cereblon), ASB1 (ankyrin repeat and SOCS box protein 1), TRIM21 (tripartite motif-containing protein 21), RNF4 (RING finger protein), UBE2D1 (ubiquitin conjugating enzyme E2 D1), UBE2D4 and ZNFR1 (Zinc and ring finger 1).

33. The fusion protein according to claim 31, wherein the fragment of E3 ubiquitin ligase or variant thereof comprises any one amino acid sequence selected from the group consisting of SEQ ID NOs: 18 to 31.

34. The fusion protein according to claim 31, wherein the fusion protein comprises a linker.

35. The fusion protein according to claim 34, wherein the fusion protein has Structural Formula (I) or (II) below:
N'-TB-(L)n-SR-C' (I)
N'-SR-(L)n-TB-C' (II)
wherein in Structural Formulas (I) and (II),
N' is an N-terminus of the fusion protein,
C' is a C-terminus of the fusion protein,
TB is an anti-SOX2 single domain antibody or an antigen-binding fragment thereof,
SR is a fragment of E3 ubiquitin ligase or a variant thereof,
L is a peptide linker, and
n is 0 or 1.

36. The fusion protein according to any one of claims 31 to 35, wherein the fusion protein comprises any one amino acid sequence selected from the group consisting of SEQ ID NOs: 164 to 184.

37. A polynucleotide, encoding the fusion protein according to any one of claims 31 to 36.

38. An expression vector, loaded with the polynucleotide according to claim 37.

39. A transformed cell, into which the expression vector according to claim 38 is introduced.

40. A method for producing a fusion protein, comprising culturing the transformed cell according to claim 39.

41. A pharmaceutical composition for preventing or treating cancer, comprising the fusion protein according to any one of claims 31 to 36; the polynucleotide according to claim 37; or the vector according to claim 38 as an active ingredient.

42. The pharmaceutical composition according to claim 41, wherein the cancer is any one selected from the group consisting of breast cancer, colon cancer, esophageal cancer, stomach cancer, liver cancer, lung cancer, pancreatic cancer, prostate cancer, gallbladder cancer, bladder cancer, kidney cancer, skin cancer, rectal cancer, osteosarcoma, multiple myeloma, glioma, ovarian cancer, cervical cancer, endometrial cancer, thyroid cancer, laryngeal cancer, testicular cancer, mesothelioma, acute myeloid leukemia, chronic myeloid leukemia, acute lymphoblastic leukemia, chronic lymphoblastic leukemia, brain tumor, neuroblastoma, retinoblastoma, head and neck cancer, salivary gland cancer and lymphoma.

43. Use of the fusion protein according to any one of claims 31 to 36; the polynucleotide according to claim 37; or the vector according to claim 38 in the prevention or treatment of cancer.

44. A method for preventing or treating cancer, comprising administering the fusion protein according to any one of claims 31 to 36; the polynucleotide according to claim 37; or the vector according to claim 38 to a subject.
